# EUROPEAN PATENT SPECIFICATION

(11) **EP 3 816 627 B1**
(45) Date of publication and mention of the grant of the patent: **29.11.2023**
(21) Application number: 19205914.5
(22) Date of filing: 29.10.2019
(51) Int. Cl.: G01N 33/574

(54) **EUKARYOTIC TRANSLATION INITIATION FACTORS (EIFS) AS NOVEL BIOMARKERS IN ENDOMETRIAL CANCER**
EUKARYOTISCHE TRANSLATIONSINITIATIONSFAKTOREN (EIFS) ALS NEUARTIGE BIOMARKER BEI ENDOMETRIUMKREBS
FACTEURS D'INITIATION DE TRADUCTION EUCARYOTE (EIFS) EN TANT QUE NOUVEAUX BIOMARQUEURS DANS LE CANCER DE L'ENDOMÈTRE

(43) Date of publication of application: 05.05.2021
(73) Proprietor: Otto-von-Guericke-Universität Magdeburg, 39106 Magdeburg (DE)
(72) Inventor: Haybäck, Johannes, 39104 Magdeburg (AT); Smolle, Maria Anna, 8047 Graz (AT)
(74) Representative: Geling, Andrea

(56) References cited:
- M. SMOLLE ET AL: "Translation initiation in endometrial carcinoma", ESP ABSTRACTS 2015, vol. 467, 5 August 2015 (2015-08-05), pages s45-s45, XP055684756,
- JIE MIN ET AL: "Eukaryotic initiation factor 3B downregulation suppresses cell proliferation, migration and invasion while it induces cell apoptosis by blocking the [beta]-catenin pathway in endometrial cancer", INT J CLIN EXP PATHOL, vol. 12, no. 9, 1 January 2019 (2019-01-01), pages 3595-3603, XP055684456,
- KAI ZHANG ET AL: "Identification of key genes and pathways between type?I and type?II endometrial cancer using bioinformatics analysis", ONCOLOGY LETTERS, 28 June 2019 (2019-06-28), XP055684447, GR ISSN: 1792-1074, DOI: 10.3892/ol.2019.10550
- SHI Z M ET AL: "Expression profile of eukaryotic translation initiation factor and matrix metalloproteinase 9 in endometrial cancer tissue.", JOURNAL OF BIOLOGICAL REGULATORS AND HOMEOSTATIC AGENTS, vol. 31, no. 4, October 2017 (2017-10), pages 1053-1059, XP009519858, ISSN: 0393-974X
- OMAR HABEEB ET AL: "Epithelial membrane protein-2 expression is an early predictor of endometrial cancer development", CANCER., vol. 116, no. 20, 19 August 2010 (2010-08-19), pages 4718-4726, XP055684901, US ISSN: 0008-543X, DOI: 10.1002/cncr.25259
- JIA-ZHEN XU ET AL: "The eIF3a Arg803Lys genetic polymorphism is associated with susceptibility to and chemoradiotherapy efficacy in cervical carcinoma", KAOHSIUNG JOURNAL OF MEDICAL SCIENCES, vol. 33, no. 4, 1 April 2017 (2017-04-01), pages 187-194, XP055684900, TW ISSN: 1607-551X, DOI: 10.1016/j.kjms.2017.01.008
- DING ZHENJIANG ET AL: "Upregulation of eukaryotic translation initiation factor 3 subunit a promotes cell survival in ameloblastoma", ORAL SURGERY, ORAL MEDICINE, ORAL PATHOLOGY AND ORAL RADIOLOGY, ELSEVIER, AMSTERDAM, NL, vol. 128, no. 2, 1 August 2019 (2019-08-01), pages 146-153, XP085732308, ISSN: 2212-4403, DOI: 10.1016/J.OOOO.2019.02.016 [retrieved on 2019-02-22]

## Description

The present invention relates to a method of diagnosing endometrial cancer in an individual. Further, the present invention relates to a method of grading endometrial cancer in an individual. Furthermore, the present invention relates to a kit useful to carry out these methods.

### BACKGROUND OF THE INVENTION

Endometrial cancer (EC) is the fifth most common cancer in females and the most frequently diagnosed gynaecological malignancy in developed countries (1). The mean age of patients at the time of diagnosis is 63 years (2). Main risk factors include diabetes, alcohol abuse, a history of infertility and elevated oestrogen levels due to obesity, polycystic ovary syndrome and oestrogen use (3). Early-stage EC is limited to the uterus and associated with a rather good prognosis. However, the increasing incidence of non-endometrioid subtypes, high-grade tumours and advanced-stage ECs at the tie of diagnosis has led to a doubling of EC-related deaths over the last 30 years (4). Historically, ECs were subdivided into two groups, based on the pathogenesis and histological presentation (5). Type I ECs arise in an oestrogen-rich environment on the basis of endometrial hyperplasia, express hormone receptors, present with a well-differentiated endometrioid histological pattern and are associated with good prognosis. On the other hand, type II ECs develop from atrophic endometrium, are not associated with oestrogen excess, have a non-endometrioid differentiation (i.e. serous, clear cell) and carry a worse prognosis compared to type I EC (5). Over the last years, characterisation of EC based on molecular features has gained significance. In parallel, the role of type I and II EC has become less important, since molecular features frequently overlap between the two types (6). Mutations in the *PIK3CA* genes are found in about half of serous and endometrioid carcinomas (7). In nearly 90% of ECs with serous differentiation, inactivating *TP53* mutations are present, and about 80% of endometrioid carcinomas harbour inactivating *PTEN* mutations (7, 8). Consequently, mutations in the *TP53* gene are thought to drive development of serous subtypes, whilst a PTEN loss is rather causative of endometrioid carcinoma (9).

The most important prognostic factor is surgical stage, followed by tumour size, grade, subtype and patients' age (10). Importantly, the addition of molecular features further aids outcome estimation (8).

Smolle M. et al. (ESP Abstracts 2015, Vol. 467, page s45) refer to translation initiation in endometrial cancer. Jie Min et al. (Int J Clin Exp Pathol, Vol. 12, No. 9, 2019, pages 3595 to 3603 describe that the downregulation of eukaryotic initiation factor 3B suppresses cell proliferation, migration and invasion while it induces cell apoptosis by blocking the [beta]-catenin pathway in endometrial cancer. Further, Kai Zhang et al. (Oncology Letters, 2019) describe the identification of key genes and pathways between type I and II endometrial cancer using bioinformatics analysis. Furthermore, Shi Z. M. et al. (Journal of Biological Regulators and Homeostatic agents, Vol. 31, No. 4, 2017, pages 1053 to 1059) describe the expression profile of eukaryotic translation initiation factor and matrix metalloproteinase 9 in endometrial cancer tissue. In addition, Omar Habeeb et al. (Cancer, Vol. 116, No. 20, 2010, pages 4718 to 4726) describe that epithelial membrane protein-2 expression is an early predictor of endometrial cancer development WO2018024609 discloses the use of eIF6 as a biomarker for hepatocellular carcinoma.

In view of the above, there is a special need for the identification of biomarkers allowing the diagnosis and/or monitoring of endometrial cancer. There is also a special need for the identification of biomarkers allwoing the grading of endometrial cancer.

In recent years, the role of eukaryotic translation initiation factors (eIFs) in carcinogenesis has been steadily uncovered. They are involved in tumour development, progression and invasion. eIFs mainly regulate the classic - or canonical - process of translation initiation and can have oncogenic or tumour-suppressive functions. Twelve core eIFs are involved in translation initiation, namely eIF1, eIF1a, eIF2, eIF2b, eIF3, eIF4h, eIF4a, eIF4e, eIF4g, eIF4b, eIF5 and eIF5b. The latter three eIFs form the heterotrimeric eIF4F complex, which mediates the ribosomal recruitment to RNA as the rate-limiting process in translation initiation. Moreover, eIFs are involved in cell cycle regulation and interact with important tumour-promoting pathways, including mTOR- and NF-kB-signalling.

The role of eukaryotic translation initiation factors (eIFs) in endometrial cancer (EC) is relatively unknown. The present inventors studied this role. 279 patients with EC were examined by the present inventors (mean age: 63.0 years; mean follow-up: 6.1 years). 1180 samples were analysed for expression of the subunits peIF2α, eIF2α, eIF3c, eIF4g, eIF5, and eIF6 through immunohistochemistry and western blotting. 15 samples of healthy endometrium served as controls. Density and intensity were assessed and mean combined scores (CS) calculated for each patient. The present inventors finally found that eIFs are deregulated between endometrial cancer and non-neoplastic tissue (NNT). In immunohistochemistry, eIF2α and eIF5 expression levels were significantly higher in tumours compared with non-neoplastic tissue (NNT) and eIF3c and eIF6 expression levels were significantly lower in tumours compared with non-neoplastic tissue (NNT). Upon western blot analysis, peIF2α and eIF4g were significantly overexpressed in EC, while expression of eIF3c was significantly reduced in EC compared with non-neoplastic tissue. Thus, eIF2α, peIF2α, eIF5, eIF6, eIF3c, and eIF4g allow the diagnosis of endometrial cancer. Further, eIF4g allows to differentiate between endometrial cancer of type I and endometrial cancer of type II. Furthermore, eIF5 and eIF6 allow the grading of endometrial cancer. These new biomarkers make a quick and accurate clinical diagnostics possible.

### SUMMARY OF THE INVENTION

In a first aspect, the present invention relates to an *in vitro* method of diagnosing endometrial cancer in an individual (suspected of having endometrial cancer) comprising the step of: determining the level of eukaryotic Initiation Factor 6 (eIF6) in a biological sample from an individual.

In a second aspect, the present invention relates to an *in vitro* method of grading endometrial cancer in an individual (suffering from endometrial cancer) comprising the step of: determining the level of eukaryotic Initiation Factor 6 (eIF6) in a biological sample from an individual.

In a third aspect, the present invention relates to the *in vitro* use of eIF6 for diagnosing endometrial cancer in an individual (suspected of having endometrial cancer).

In a fourth aspect, the present invention relates to the *in vitro* use of eIF6 for grading endometrial cancer in an individual (suffering from endometrial cancer).

In a fifth aspect, the present invention relates to the use of a kit for diagnosing or grading endometrial cancer in an individual, wherein said kit comprises means for determining the level of eIF6 in a biological sample from the individual.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

The term "comprise" or variations such as "comprises" or "comprising" according to the present invention means the inclusion of a stated integer or group of integers but not the exclusion of any other integer or group of integers. The term "consisting essentially of" according to the present invention means the inclusion of a stated integer or group of integers, while excluding modifications or other integers which would materially affect or alter the stated integer. The term "consisting of" or variations such as "consists of" according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The term "endometrial cancer (EC)", as used herein, refers to a cancer that arises from the endometrium (the lining of the uterus or womb). It is the result of the abnormal growth of cells that have the ability to invade or spread to other parts of the body. The first sign is most often vaginal bleeding not associated with a menstrual period. Other symptoms include pain with urination, pain during sexual intercourse or pelvic pain. Endometrial cancer occurs most commonly after menopause. Endometrial cancer comprises, but is not limited to, endometrioid cancer, uterine serous carcinomas, and clear cell carcinomas. Endometrial cancer can further be distinguished into two main types, namely endometrial cancer of type I (EC I) and endometrial cancer of type II (EC II). Endometrial cancer of type I encompasses endometrioid cancer of grades 1 and 2, and endometrial cancer of type II encompasses non-endometrioid carcinomas such as uterine serous carcinomas and clear cell carcinomas as well as endometrioid cancer of grade 3 (depending on pathogenesis).

The term "endometrial cancer of type I", as used herein, refers to cancer which is estrogen dependent. Endometrial cancer of type I is the most common type (80% to 90% of all endometrial cancer). Endometrial cancer of type I is generally slow growing and less likely to spread. In addition, it is often preceded by endometrial hyperplasia. Many established risk factors for endometrial cancer of type I are related to an imbalance between estrogen and progesterone exposures, including obesity and the use of unopposed estrogen therapy. Use of combined oral contraceptives (OCs), which is associated with progesterone-dominant states, reduces the risk of endometrial cancer. Other risk factors include nulliparity, early menarche, and late menopause, whereas smoking is associated with reduced risk. Endometrial cancer of type I usually encompasses endometrial cancer of grades 1 and 2. In particular, endometrial cancer of type I encompasses endometrioid cancer of grades 1 and 2.

The term "endometrioid cancer", as used herein, refers to a cancer which is pronounced endo-mee-tree-oyd. This cancer is often diagnosed at an early stage and so is usually treated successfully. More than 3 out of 4 adenocarcinomas of the womb lining are this type. An endometrioid carcinoma is composed of cells that resemble the normal endometrium and can be associated with or preceded by the abnormal multiplication of normal cells of the endometrium, a phenomenon called endometrial hyperplasia. There are different subtypes of endometrioid cancer. Some types have squamous cells as well as glandular cells. Adenoacanthomas have a mix of cancerous glandular cells and non-cancerous (benign) squamous cells. If both the glandular cells and squamous cells are cancerous, it is called adenosquamous carcinoma.

The term "endometrial cancer of type II", as used herein, refers to cancer which is estrogen independent. Endometrial cancer of type II is predominantly serous. It arises in atrophic endometrium and derives from intraepithelial carcinoma, a precancerous lesion. Endometrial cancer of type II is generally less well differentiated and has poorer prognoses than type I EC cancer. Endometrial cancer of type II accounts for a disproportionate number of endometrial cancer deaths (40% of deaths, whereas they only account for 10% to 20% of cases). Little is known about risk factors for endometrial cancer of type II, mainly because most epidemiologic studies have lacked enough cases to study these less common tumors separately. Endometrial cancer of type II usually encompasses endometrial cancer of grade 3. In particular, endometrial cancer of type II encompasses uterine serous carcinomas, clear cell carcinomas, and endometrioid cancer of grade 3.

The term "uterine serous carcinoma", as used herein, refers to cancer which is not hormonally mediated and arises from endometrial intraepithelial carcinoma, a recognized precursor lesion. Uterine serous carcinoma is considered high-grade with marked nuclear pleomorphism, multinucleated cells, psammoma bodies, hobnail cells, uneven gland borders, and papillary or solid growth patterns with slitlike spaces. It is much less common than endometrioid cancer. Only about 5 out of every 100 womb cancers (5%) are the uterine serous type. This is a more quickly growing type of cancer that is more likely to come back than other types, even if it is caught early.

The term "clear cell carcinoma", as used herein, refers to cancer which is very rare. These days, only about 1 or 2 cases of womb cancer in every 100 (1 to 2%) are clear cell cancer.

The term "diagnosing endometrial cancer", as used herein, means determining whether an individual shows signs of or suffers from endometrial cancer.

The term "determining the course of endometrial cancer", as used herein, means determining the development of endometrial cancer over time, e.g. whether endometrial cancer worsens in the individual, does not worsen/is stable in the individual, or improves in the individual over time.

The term "grading endometrial cancer", as used herein, means determining the stage/grade of endometrial cancer in an individual suffering from endometrial cancer. In particular, it allows to determine whether the individual suffers from endometrial cancer of grade/stage 1, 2, or 3.

The term "differentiating between low-grade, median-grade, and/or high-grade endometrial cancer" means determining whether an individual suffers from low-grade endometrial cancer (e.g. endometrial cancer of grade I), median-grade endometrial cancer (e.g. endometrial cancer of grade II) or high-grade endometrial cancer (e.g. endometrial cancer of grade III).

The term "low-grade endometrial cancer", as used herein, means that the cancer cells are low grade or well differentiated. They look almost like normal cells. Low grade cancer cells tend to grow slow and are less likely to spread than higher grade cancer cells. Endometrial cancer of grade 1 belongs to low-grade endometrial cancer (see also staging below).

The term "median-grade endometrial cancer", as used herein, means that the cancer cells look more abnormal and are more likely to spread. This grade is also called moderately differentiated or moderate grade. Endometrial cancer of grade 2 belongs to median-grade endometrial cancer (see also staging below).

The term "high-grade endometrial cancer", as used herein, means that the cancer cells are very abnormal and not like normal cells. They tend to grow quickly and are more likely to spread. They are called poorly differentiated or high grade. Endometrial cancer of grade 3 belongs to high-grade endometrial cancer (see also staging below).

Knowing the grade of endometrial cancer allows to determine how quickly the cancer may be growing and how likely it is to spread. The grade can also help to predict future outcomes (prognosis) and how the cancer might respond to treatment.

The different grades/stages of endometrial cancer can be described as follows:
Grade/Stage 1: The cells look very like normal cells. They are also called low grade or well differentiated. They tend to be slow growing and are less likely to spread than higher grade cancer cells.
Grade/Stage 2: The cells look more abnormal and are more likely to spread. This grade is also called moderately differentiated or moderate grade.
Grade/Stage 3: The cells look very abnormal and not like normal cells. They tend to grow quickly and are more likely to spread. They are called poorly differentiated or high grade.

The terms "stage" and "grade" are interchangeably used herein.

When the histological type of endometrial cancer is endometrioid, the grade can be 1, 2 or 3. When the histological type is uterine serous or clear cell carcinoma, the grade is usually 3 and carries a worse prognosis.

The term "individual", as used herein, refers to any subject for whom it is desired to know whether she or he suffers from endometrial cancer. In particular, the term "individual", as used herein, refers to a subject suspected to be affected by endometrial cancer. The individual may be diagnosed to be affected by endometrial cancer, i.e. diseased, or may be diagnosed to be not affected by endometrial cancer, i.e. healthy. The term "individual", as used herein, also refers to a subject which is affected by endometrial cancer, i.e. diseased. The patient may be retested for endometrial cancer and may be diagnosed to be still affected by endometrial cancer, i.e. diseased, or not affected by endometrial cancer anymore, i.e. healthy, for example after therapeutic intervention. The individual may also be retested for endometrial cancer and may be diagnosed as having developed an advanced form of endometrial cancer (e.g. endometrial cancer of grade 1, endometrial cancer of grade 2 or endometrial cancer of grade 3 as well as low-grade endometrial cancer, median-grade endometrial cancer or high-grade endometrial cancer). In addition, it may be determined whether the patient suffers from endometrial cancer of type I or endometrial cancer of type II (differential diagnosis).

It should be noted that an individual that is diagnosed as being healthy, i.e. not suffering from endometrial cancer, may possibly suffer from another disease or condition not tested/known. The individual may be any mammal, including both a human and another mammal, e.g. an animal such as a rabbit, mouse, rat, or monkey. Human individuals are particularly preferred.

The term "(control) patient", as used herein, refers to a subject known to be affected by endometrial cancer, i.e. diseased. Said (control) patient may have developed an advanced form of endometrial cancer. For example, the (control) patient is a (control) patient with endometrial cancer of a specific grade (e.g. grade 1, 2, or 3). The (control) patient may also be a (control) patient suffering from low-grade endometrial cancer, median-grade endometrial cancer, or high-grade endometrial cancer. The (control) patient may further be a (control) patient suffering from endometrial cancer of type I or from endometrial cancer of type II.
The (control) patient may be any mammal, including both a human and another mammal, e.g. an animal such as a rabbit, mouse, rat, or monkey. Human (control) patients are particularly preferred.

The term "healthy (control) individual", as used herein, refers to a subject known to be not affected by endometrial cancer (negative control), i.e. healthy.
It should be noted that an individual which is known to be healthy, i.e. not suffering from endometrial cancer, may possibly suffer from another disease or condition not tested/known. The healthy individual may be any mammal, including both a human and another mammal, e.g. an animal such as a rabbit, mouse, rat, or monkey. Human healthy individuals are particularly preferred.

The term "treatment", in particular "therapeutic treatment", as used herein, refers to any therapy which improves the health status and/or prolongs (increases) the lifespan of an individual suffering from a disease or condition, in particular a tumor. Said therapy may eliminate the disease or condition in an individual, arrest or slow the development of a disease in an individual, inhibit or slow the development of a disease in an individual, decrease the frequency or severity of symptoms in an individual, and/or decrease the recurrence in an individual who currently has or who previously has had a disease. The treatment of endometrial cancer is preferably selected from the group consisting of the administration of a drug, surgery, chemotherapy, radiotherapy, and a combination thereof.

The term "level", as used herein, refers to an amount (measured for example in grams, mole, or ion counts) or concentration (e.g. absolute or relative concentration) of the at least one elF claimed herein. The term "level", as used herein, also comprises scaled, normalized, or scaled and normalized amounts or values. The level may also be a cut-off level. In one embodiment, the level is an expression level.

The term "eukaryotic Initiation Factor (eIF)", as used herein, refers to molecules which are involved in the initiation phase of eukaryotic translation. These factors help to stabilize the formation of the functional ribosome around the start codon and also provide regulatory mechanisms in translation initiation. The following eIFs are mentioned herein: peIF2α, eIF2α, eIF3c, eIF4g, eIF5, and eIF6. The term "eukaryotic Initiation Factor (eIF)", as used herein, covers elF RNA transcripts (RNA transcript variants) such as mRNAs including splice variants of these transcripts and elF proteins encoded thereby. Thus, the level of the eIFs may be determined by measuring mRNA or protein levels. The term "eukaryotic Initiation Factor (eIF)", as used herein, also covers elF isoforms. These eIF isoforms are members of a set of highly similar molecules, in particular proteins, that perform the same or similar biological role. For example, eIF4g comprises/encompasses the isoforms eIF4g1, eIF4g2, and/or eIF4g3, encoded by the respective genes. The level of elF isoforms may also be determined by measuring mRNA or protein levels. Thus, when it is referred to eIF4g herein, also the isoforms eIF4g1, eIF4g2, and eIF4g3 are meant.

The term "biological sample", as used herein, refers to any biological sample from an individual or (control) patient comprising at least one of the eIFs claimed herein. The biological sample may be a body fluid sample, e.g. a blood sample or urine sample, or a tissue sample. Biological samples may be mixed or pooled, e.g. a sample may be a mixture of a blood sample and a urine sample. Said biological samples may be provided by removing a body fluid from an individual or (control) patient, but may also be provided by using a previously isolated sample. For example, a blood sample may be taken from an individual or (control) patient by conventional blood collection techniques. The biological sample, e.g. urine sample or blood sample, may be obtained from an individual or (control) patient prior to the initiation of a therapeutic treatment, during the therapeutic treatment, and/or after the therapeutic treatment. If the biological sample, is obtained from at least one (control) patient or healthy (control) individual, e.g. from at least 1, 2, 3, 4, 5, 6, 7, 8, 9, 10, 20, 30, 40, 50, 60, 70, 80, 90, 100, 150, 200, 250, 300, 400, 500, or 1.000 (control) patient(s) or healthy (control) individual(s), it is designated as a "reference biological sample". Preferably, the reference biological sample is from the same source than the biological sample of the individual to be tested, e.g. both are blood samples or urine samples. It is further preferred that both are from the same species, e.g. from a human. It is also (alternatively or additionally) preferred that the measurements of the reference biological sample and the biological sample of the individual to be tested are identical, e.g. both have an identical volume. It is particularly preferred that the reference biological sample and the biological sample are from individuals/(control) patients of the same sex and similar age, e.g. no more than 2 years apart from each other.

The term "body fluid sample", as used herein, refers to any liquid sample derived from the body of an individual or (control) patient containing at least one of the eIFs claimed herein. Said body fluid sample may be a urine sample, blood sample, sputum sample, breast milk sample, cerebrospinal fluid (CSF) sample, cerumen (earwax) sample, gastric juice sample, mucus sample, lymph sample, endolymph fluid sample, perilymph fluid sample, peritoneal fluid sample, pleural fluid sample, saliva sample, sebum (skin oil) sample, semen sample, sweat sample, tears sample, cheek swab, vaginal secretion sample, liquid biopsy, or vomit sample including components or fractions thereof. The term "body fluid sample" also encompasses body fluid fractions, e.g. blood fractions, urine fractions or sputum fractions. Body fluid samples may be mixed or pooled. Thus, a body fluid sample may be a mixture of a blood and a urine sample or a mixture of a blood and cerebrospinal fluid sample. Said body fluid sample may be provided by removing a body liquid from an individual or (control) patient, but may also be provided by using previously isolated body fluid sample material. The body fluid sample allows for a non-invasive analysis of an individual. It is further preferred that the body fluid sample has a volume of between 0.01 and 20 ml, more preferably of between 0.1 and 10 ml, even more preferably of between 0.5 and 8 ml, and most preferably of between 1 and 5 ml.

The term "blood sample", as used herein, encompasses a whole blood sample or a blood fraction sample such as a blood serum or blood plasma sample. It is preferred that the blood serum or plasma sample has a volume of between 0.01 and 20 ml, more preferably of between 0.1 and 10 ml, even more preferably of between 0.5 and 8 ml and most preferably of between 1 and 5 ml.

In the context of the present invention, the term "kit of parts (in short: kit)" is understood to be any combination of at least some of the components identified herein, which are combined, coexisting spatially, to a functional unit, and which can contain further components. Said kit may allow point-of-care testing (POCT).

The term "point-of-care testing (POCT)", as used herein, refers to a medical diagnostic testing at or near the point of care that is the time and place of individual care. This contrasts with the historical pattern in which testing was wholly or mostly confined to the medical laboratory, which entailed sending off specimens away from the point of care and then waiting hours or days to learn the results, during which time care must continue without the desired information. Point-of-care tests are simple medical tests that can be performed at the bedside. The driving notion behind POCT is to bring the test conveniently and immediately to the individual to be tested. This increases the likelihood that the individual, physician, and care team will receive the results quicker, which allows for immediate clinical management decisions to be made. POCT is often accomplished through the use of transportable, portable, and handheld instruments and test kits. Small bench analyzers or fixed equipment can also be used when a handheld device is not available - the goal is to collect the specimen and obtain the results in a very short period of time at or near the location of the individual so that the treatment plan can be adjusted as necessary before the individual leaves the hospital.

### Embodiments of the invention

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous, unless clearly indicated to the contrary.

The role of eukaryotic translation initiation factors (eIFs) in endometrial cancer (EC) is relatively unknown. The present inventors studied this role. 279 patients with EC were examined by the present inventors (mean age: 63.0 years; mean follow-up: 6.1 years). 1180 samples were analysed for expression of the subunits peIF2α, eIF2α, eIF3c, eIF4g, eIF5, and eIF6 through immunohistochemistry and western blotting. 15 samples of healthy endometrium served as controls. Density and intensity were assessed and mean combined scores (CS) calculated for each patient. The present inventors finally found that eIFs are deregulated between endometrial cancer and non-neoplastic tissue (NNT). In immunohistochemistry, eIF2α and eIF5 expression levels were significantly higher in tumours compared with non-neoplastic tissue (NNT) and eIF3c and eIF6 expression levels were significantly lower in tumours compared with non-neoplastic tissue (NNT). Upon western blot analysis, peIF2α and eIF4g were significantly overexpressed in EC, while expression of eIF3c was significantly reduced in EC compared with non-neoplastic tissue. Thus, eIF2α, peIF2α, eIF5, eIF6, eIF3c, and eIF4g allow the diagnosis of endometrial cancer. Further, eIF4g allows to differentiate between endometrial cancer of type I and endometrial cancer of type II. Furthermore, eIF5 and eIF6 allow the grading of endometrial cancer. These new biomarkers make a quick and accurate clinical diagnostics possible.

Thus, in a first aspect, the present invention relates to a (an *in vitro)* method of diagnosing endometrial cancer in an individual (suspected of having endometrial cancer) comprising the step of:
determining the level of eukaryotic Initiation Factor 6 (eIF6) in a biological sample from an individual.

In particular, said individual is suspected of suffering from endometrial cancer.

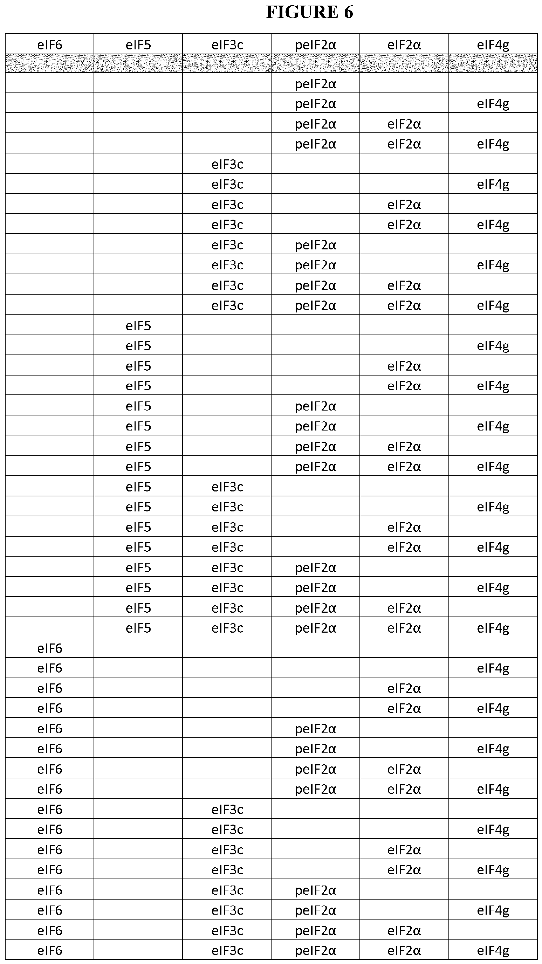

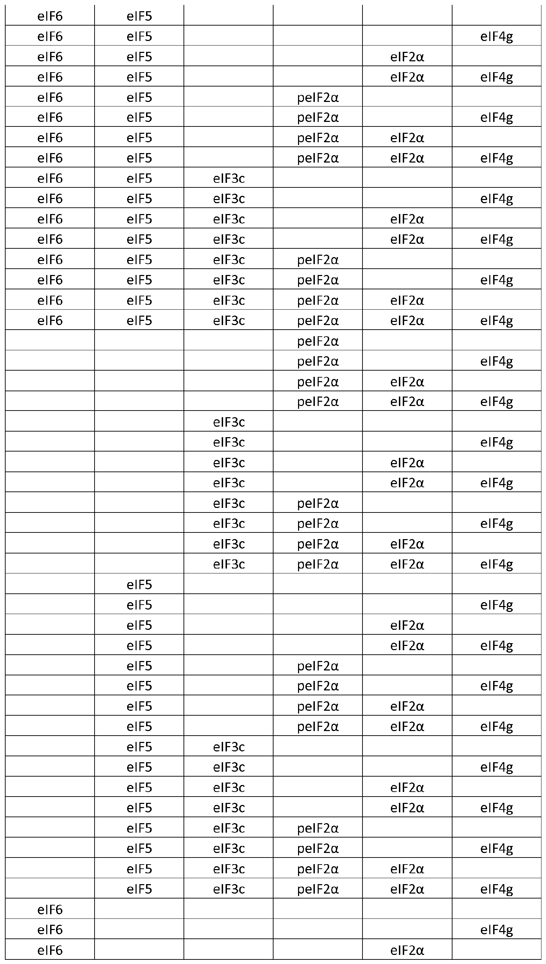

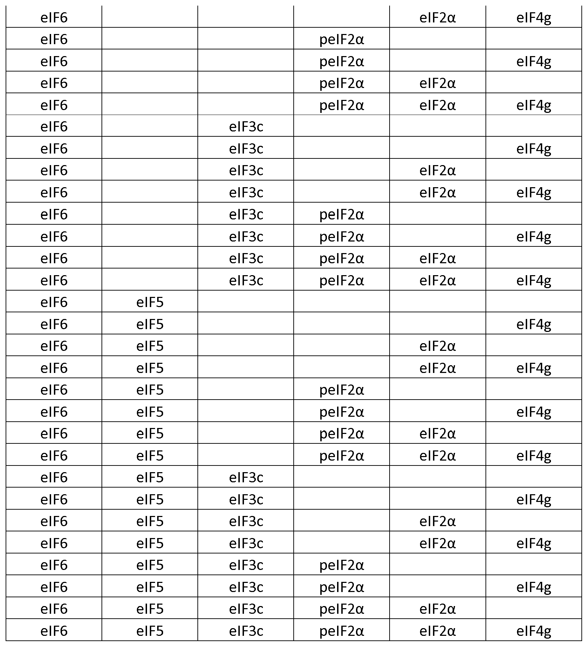

Preferred combinations with eIF6 can be taken from **Figure 6****.**

In one embodiment, the level of eIF6 is compared to a reference level of said eIF6. Thus, in one particular embodiment, the present invention relates to a method of diagnosing endometrial cancer in an individual (suspected of suffering from endometrial cancer) comprising the steps of:
(i) determining the level of eukaryotic Initiation Factor 6 (eIF6) in a biological sample from an individual, and
(ii) comparing the level of eIF6 to a reference level of said eIF6.

The above comparison allows to diagnose endometrial cancer in an individual, in particular in an individual suspected of having endometrial cancer. The individual may be diagnosed as suffering from endometrial cancer, i.e. being diseased, or as not suffering from endometrial cancer, i.e. being healthy.

The reference level may be any level which allows to determine whether an individual suffers from endometrial cancer or not. It may be obtained from a (control) subject (i.e. a subject different from the individual to be tested such as a healthy individual and/or a patient having endometrial cancer) or from the same individual. In the latter case, the individual may be retested for endometrial cancer, e.g. in the form of a longitudinal monitoring. It may be determined that the individual is now affected by endometrial cancer or still not affected by endometrial cancer.

It is preferred that the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference biological sample(s), from at least one healthy individual, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 healthy individual(s). It is more preferred that the reference level is the level determined by measuring between 2 and 500 reference biological samples from between 2 and 500 healthy individuals. It is even more preferred that the reference level is determined by measuring between 50 and 500 reference biological samples from between 50 and 500 healthy individuals. It is most preferred that the reference level is determined by measuring between 100 and 500 reference biological samples from between 100 and 500 healthy individuals.

It is practicable to take one reference biological sample per individual for analysis. If additional reference biological samples are required, e.g. to determine the reference level in different reference biological samples, the same individual may be (re)tested. Said reference level may be an average reference level. It may be determined by measuring reference levels and calculating the "average" value (e.g. mean, median or modal value) thereof.

It is further preferred that the level of eIF6 which is below the reference level of said eIF6 indicates that the individual suffers from endometrial cancer.

Preferably, the level of eIF6 is at least 0.6-fold or 0.7-fold, more preferably at least 0.8-fold or 0.9-fold, even more preferably at least 1.2-fold or 1.5-fold, and most preferably at least 2.0-fold or 3.0-fold below the reference level. For example, the level of eIF6 is at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2. 1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below the reference level.

In one another embodiment, the level of at least one further elF is determined in the biological sample from an individual,
wherein the at least one further eIF is selected from the group consisting of eIF4g and eIF2α.

It should be noted that eIF4G preferably comprises the isoforms eIF4g1, eIF4g2, and/or eIF4g3.

For example, the level of eIF4g, the level of eIF2α, or the level of eIF4g and the level of eIF2α is (are) further determined.

Preferred combinations with eIF6 can be taken from **Figure 6****.**

It is preferred that the level of the at least one further elF is compared to a reference level of said at least one further eIF. Thus, in one particular embodiment, the present invention relates to a method of diagnosing endometrial cancer in an individual (suspected of suffering from endometrial cancer) comprising the steps of:
(i) determining the level of eukaryotic Initiation Factor 6 (eIF6) in a biological sample from an individual and determining the level of at least one further eIF, e.g. 1 or 2 eIF(s), in the biological sample from an individual, wherein the at least one further elF is selected from the group consisting of eIF4g and eIF2α, and
(ii) comparing the level of eIF6 to a reference level of said eIF6 and the level of the at least one further eIF to a reference level of said at least one further elF.

It is also preferred that the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference biological sample(s), from at least one healthy individual, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 healthy individual(s). It is more preferred that the reference level is the level determined by measuring between 2 and 500 reference biological samples from between 2 and 500 healthy individuals. It is even more preferred that the reference level is determined by measuring between 50 and 500 reference biological samples from between 50 and 500 healthy individuals. It is most preferred that the reference level is determined by measuring between 100 and 500 reference biological samples from between 100 and 500 healthy individuals.

It is further preferred that
the level of eIF4g which is above the reference level indicates that the individual suffers from endometrial cancer, and/or
the level of eIF2α which is above the reference level indicates that the individual suffers from endometrial cancer.

Preferably, the level of the at least one elF is at least 0.6-fold or 0.7-fold, more preferably at least 0.8-fold or 0.9-fold, even more preferably at least 1.2-fold or 1.5-fold, and most preferably at least 2.0-fold or 3.0-fold above the reference level. For example, the level of the at least one elF is at least 0.6-fold, at least 0.7-fold, at least 0.8-fold, at least 0.9-fold, at least 1.0-fold, at least 1. 1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold above the reference level.

Preferably, the endometrial cancer is selected from the group consisting of endometrioid cancer, uterine serous carcinomas, and clear cell carcinomas. Endometrial cancer can further be distinguished into two main types, namely endometrial cancer of type I (EC I) and endometrial cancer of type II (EC II). Thus, it is further preferred that the endometrial cancer is endometrial cancer of type I or endometrial cancer of type II. More preferably, the endometrial cancer of type I is endometrioid cancer of grades 1 and 2. More preferably, the endometrial cancer of type II is uterine serous carcinomas, clear cell carcinomas or endometrioid cancer of grade 3.

The present inventors further found that eIF5 allows to determine the course of endometrial cancer in an individual (suffering from endometrial cancer). Further described herein, but not encompassed by the present invention, is a (an *in vitro)* method of determining the course of endometrial cancer in an individual (suffering from endometrial cancer) comprising the step of: determining the level of the eukaryotic Initiation Factor (eIF) eIF5 in a biological sample from an individual.

In particular, said individual suffers from endometrial cancer.

It one embodiment said determining comprises determining the level of eIF5 in a biological sample at a first point in time and in at least one further biological sample at a later point in time and comparing said levels determined at the different time points. Thus, in one particular embodiment, a method of determining the course of endometrial cancer in an individual (suffering from endometrial cancer) comprising the steps of:
(i) determining the level of eIF5 in a biological sample from an individual (suffering from endometrial cancer) at a first point in time and in at least one further biological sample from the (same) individual at a later point in time, and
(ii) comparing said levels determined at the different time points is described.
This proceeding allows to determine the course of endometrial cancer in an individual suffering from endometrial cancer over an extended period of time, such as months or years, e.g. 1, 2, 3, 4, 5, 6, 7, 8, 9, 10, or 11 month(s), 1, 2, 3, 4, or 5 year(s).

It is preferred that the level of eIF5 which
(i) increases over time indicates that endometrial cancer worsens in the individual,
(ii) does not change over time indicates that endometrial cancer does not worsen/is stable in the individual, or
(iii) decreases over time indicates that endometrial cancer improves in the individual.

The increase may be at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2. 1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold over time.

The decrease may be at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2. 1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold over time.

"Stable", as mentioned above, means that the level varies over time between 0 and < 20%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Stable" in this respect may also mean that the detected level variation is within the accuracy of a measurement. The accuracy of a measurement depends on the measurement method used. Preferably, the level is constant over time.

The time period between the first point in time and the later point(s) in time preferably amounts to at least 1 day, at least 2 days, at least 3 days, at least 4 days, at least 5 days, at least 6 days, at least 7 days (1 week), at least 2 weeks, at least 3 weeks, at least 4 weeks, at least 1 month, at least 2 months, at least 3 months, at least 4 months, at least 5 months, at least 6 months, at least 7 months, at least 8 months, at least 9 months, at least 10 months, at least 11 months, at least 12 months (1 year), at least 24 months (2 years), at least 3 years, at least 4 years, at least 5 years, at least 6 years, at least 7 years, at least 8 years, at least 9 years, or at least 10 years. For example, the individual may be routinely checked, e.g. once or twice a year. The individual may be (re)tested at 2, 3, 4, 5, 6 7, 8, 9, or 10 time points (first point in time and further point(s) in time).

In addition to the determination of the course of endometrial cancer, the treatment of this disease can be monitored. In particular, the individual receives, has received, or had received a therapeutic treatment of endometrial cancer.

It is more preferred that
(i) the level of eIF5 which decreases over time indicates that the individual responds to said treatment,
(ii) the level of eIF5 which does not change over time indicates that the individual does not respond to said treatment, or
(iii) level of eIF5 which increases over time indicates that the individual does not respond to said treatment.

A treatment response which is not sufficient is a response which does not improve the health status of the individual.

The therapeutic treatment of endometrial cancer is preferably selected from the group consisting of the administration of a drug, surgery, chemotherapy, radiotherapy, and a combination thereof.

The individual may receive a treatment during the complete determination/monitoring process (e.g. the administration of a drug) or may receive a treatment before, at, or after a first point in time (e.g. the administration of a drug) and may be retested at a later point in time. In particular, said first point in time may be before the initiation of a treatment and said later point in time may be during the treatment and/or after the treatment. If the treatment encompasses the administration of a drug and the individual responds to said treatment, the drug administration may be continued, the dose of the drug may be reduced, or the drug administration may be stopped. If the treatment encompasses the administration of a drug and the individual does not respond to said treatment, the dose of the drug may be increased, the drug may be changed, or the therapy mode may be changed, e.g. from drug administration to surgery or radiotherapy.

Preferably, the endometrial cancer is selected from the group consisting of endometrioid cancer, uterine serous carcinomas, and clear cell carcinomas. Endometrial cancer can further be distinguished into two main types, namely endometrial cancer of type I (EC I) and endometrial cancer of type II (EC II). Thus, it is further preferred that the endometrial cancer is endometrial cancer of type I or endometrial cancer of type II. More preferably, the endometrial cancer of type I is endometrioid cancer of grades 1 and 2. More preferably, the endometrial cancer of type II is uterine serous carcinomas, clear cell carcinomas or endometrioid cancer of grade 3.

The present inventors further found that eIF5 and eIF6 allow grading of endometrial cancer. In a second aspect, the present invention relates to a (an *in vitro)* method of grading/staging endometrial cancer in an individual (suffering from endometrial cancer) comprising the step of:
determining the level of eukaryotic Initiation Factor 6 (eIF6) in a biological sample from an individual.

For example, the level of eIF6 is determined or the level of eIF6 and the level of eIF5 are determined.

In one embodiment, the level of eIF6 is compared to a reference level of said eIF6. Thus, in one particular embodiment, the present invention relates to a method of grading/staging endometrial cancer in an individual (suffering from endometrial cancer) comprising the steps of:
(i) determining the level of eukaryotic Initiation Factor 6 (eIF6) in a biological sample from an individual, and
(ii) comparing the level of eIF6 to a reference level of said eIF6.

The above comparison allows to grade/stage endometrial cancer in an individual, in particular in an individual suffering from endometrial cancer. It may be determined that the individual suffers from endometrial cancer of grade 1, 2, or 3.

The reference level may be any level which allows grading/staging of endometrial cancer in an individual.

It is preferred that the reference level is the level determined by measuring at least one reference biological sample
from at least one healthy individual,
from at least one patient with endometrial cancer of grade 1,
from at least one patient with endometrial cancer of grade 2, and/or
from at least one patient with endometrial cancer of grade 3.

In particular, it is preferred that the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference biological sample(s),
from at least one healthy individual, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 healthy individual(s),
from at least one patient with endometrial cancer of grade 1, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 patient(s) with endometrial cancer of grade 1,
from at least one patient with endometrial cancer of grade 2, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 patient(s) with endometrial cancer of grade 2, and/or
from at least one patient with endometrial cancer of grade 3, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 patient(s) with endometrial cancer of grade 3.

Preferably, the reference level is the level determined by measuring between 2 and 500 reference biological samples from between 2 and 500 healthy individuals, patients with endometrial cancer of grade 1, patients with endometrial cancer of grade 2, and/or patients with endometrial cancer of grade 3. More preferably, the reference level is determined by measuring between 50 and 500 reference biological samples from between 50 and 500 healthy individuals, patients with endometrial cancer of grade 1, patients with endometrial cancer of grade 2, and/or patients with endometrial cancer of grade 3. Even more preferably, the reference level is determined by measuring between 100 and 500 reference biological samples from between 100 and 500 healthy individuals, patients with endometrial cancer of grade 1, patients with endometrial cancer of grade 2, and/or patients with endometrial cancer of grade 3.

It is practicable to take one reference biological sample per individual for analysis. If additional reference biological samples are required, e.g. to determine the reference level in different reference biological samples, the same individual may be (re)tested. Said reference level may be an average reference level. It may be determined by measuring reference levels and calculating the "average" value (e.g. mean, median or modal value) thereof.

It is further preferred that
(i) the reference level of said eIF6 is the level determined by measuring at least one reference biological sample from at least one healthy individual,
   wherein the level of eIF6 below the reference level of said eIF6 indicates that the individual has endometrial cancer of grade 1,
(ii) the reference level of said eIF6 is the level determined by measuring at least one reference biological sample from at least one patient with endometrial cancer of grade 2,
   wherein the level of eIF6 above the reference level of said eIF6 indicates that the individual has endometrial cancer of grade 1, and/or
(iii) the reference level of said eIF6 is the level determined by measuring at least one reference biological sample from at least one patient with endometrial cancer of grade 3,
   wherein the level of eIF6 below the reference level of said eIF6 indicates that the individual has endometrial cancer of grade 1.

It is more preferred that
the reference level of said eIF6 is the level determined by measuring at least one reference biological sample from at least one patient with endometrial cancer of grade 2 and the reference level of said eIF6 is the level determined by measuring at least one reference biological sample from at least one patient with endometrial cancer of grade 3, wherein
the level of eIF6 above the reference level of said eIF6 determined by measuring at least one reference biological sample from at least one patient with endometrial cancer of grade 2 and the level of eIF6 below the reference level of said eIF6 determined by measuring at least one reference biological sample from at least one patient with endometrial cancer of grade 3 indicates that the individual has endometrial cancer of grade 1.

It is further preferred that
(i) the reference level of said eIF6 is the level determined by measuring at least one reference biological sample from at least one healthy individual,
   wherein the level of eIF6 below the reference level of said eIF6 indicates that the individual has endometrial cancer of grade 2,
(ii) the reference level of said eIF6 is the level determined by measuring at least one reference biological sample from at least one patient with endometrial cancer of grade 1,
   wherein the level of eIF6 below the reference level of said eIF6 indicates that the individual has endometrial cancer of grade 2, and/or
(iii) the reference level of said eIF6 is the level determined by measuring at least one reference biological sample from at least one patient with endometrial cancer of grade 3,
   wherein the level of eIF6 below the reference level of said eIF6 indicates that the individual has endometrial cancer of grade 2.

It is more preferred that
the reference level of said eIF6 is the level determined by measuring at least one reference biological sample from at least one patient with endometrial cancer of grade 1, wherein the level of eIF6 below the reference level of said eIF6 indicates that the individual has endometrial cancer of grade 2.

It is further preferred that
(i) the reference level of said eIF6 is the level determined by measuring at least one reference biological sample from at least one healthy individual,
   wherein the level of eIF6 below the reference level of said eIF6 indicates that the individual has endometrial cancer of grade 3,
(ii) the reference level of said eIF6 is the level determined by measuring at least one reference biological sample from at least one patient with endometrial cancer of grade 1,
   wherein the level of eIF6 above the reference level of said eIF6 indicates that the individual has endometrial cancer of grade 3, and/or
(iii) the reference level of said eIF6 is the level determined by measuring at least one reference biological sample from at least one patient with endometrial cancer of grade 2,
   wherein the level of eIF6 above the reference level of said eIF6 indicates that the individual has endometrial cancer of grade 3.

It is more preferred that
the reference level of said eIF6 is the level determined by measuring at least one reference biological sample from at least one healthy individual and the reference level of said eIF6 is the level determined by measuring at least one reference biological sample from at least one patient with endometrial cancer of grade 1, wherein
the level of eIF6 below the reference level of said eIF6 determined by measuring at least one reference biological sample from at least one healthy individual and the level of eIF6 above the reference level of said eIF6 determined by measuring at least one reference biological sample from at least one patient with endometrial cancer of grade 1 indicates that the individual has endometrial cancer of grade 3.

Preferably, the level of the at least one elF is at least 0.6-fold or 0.7-fold, more preferably at least 0.8-fold or 0.9-fold, even more preferably at least 1.2-fold or 1.5-fold, and most preferably at least 2.0-fold or 3.0-fold below/above the reference level. For example, the level of the at least one elF is at least 0.6-fold, at least 0.7-fold, at least 0.8-fold, at least 0.9-fold, at least 1.0-fold, at least 1. 1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2. 1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

Preferably, the endometrial cancer is selected from the group consisting of endometrioid cancer, uterine serous carcinomas, and clear cell carcinomas. Endometrial cancer can further be distinguished into two main types, namely endometrial cancer of type I (EC I) and endometrial cancer of type II (EC II). Thus, it is further preferred that the endometrial cancer is endometrial cancer of type I or endometrial cancer of type II. More preferably, the endometrial cancer of type I is endometrioid cancer of grades 1 and 2. More preferably, the endometrial cancer of type II is uterine serous carcinomas, clear cell carcinomas or endometrioid cancer of grade 3.

When the histological type of endometrial cancer is endometrioid, the grade may be 1, 2 or 3. When the histological type is uterine serous or clear cell carcinoma, the grade is usually 3 and carries a worse prognosis. Thus, it may be determined that the individual suffers from endometrioid cancer of grades 1, 2, or 3. It may also be determined that the individual suffers from uterine serous or clear cell carcinoma of grade 3.

The present inventors also found that eIF4g allows to differentiate between endometrial cancer of type I and endometrial cancer of type II in an individual. Further described herein, but not encompassed by the present invention, is a (an *in vitro)* method of differentiating between endometrial cancer of type I and endometrial cancer of type II in an individual (suffering from endometrial cancer) comprising the step of:
determining the level of the eukaryotic Initiation Factor (eIF) eIF4g in a biological sample from an individual.

In this respect, it should be noted that eIF4g preferably comprises the isoforms eIF4g1, eIF4g2, and/or eIF4g3.

In particular, said individual suffers from endometrial cancer.

In one embodiment, the level of eIF4g is compared to a reference level of eIF4g. Thus, in one particular embodiment, a method of differentiating between endometrial cancer of type I and endometrial cancer of type II in an individual (suffering from endometrial cancer) comprising the steps of:
(i) determining the level of the eukaryotic Initiation Factor (eIF) eIF4g in a biological sample from an individual, and
(ii) comparing the level of eIF4g to a reference level of eIF4g is described.

The above comparison allows to differentiate between endometrial cancer of type I and endometrial cancer of type II in an individual. It may be determined that the individual suffers from endometrial cancer of type I or from endometrial cancer of type II.

The reference level may be any level which allows to differentiate between endometrial cancer of type I and endometrial cancer of type II in an individual.

It is preferred that the reference level is the level determined by measuring at least one reference biological sample from
at least one patient with endometrial cancer of type I and/or
at least one patient with endometrial cancer of type II.

It is particularly preferred that the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference biological sample(s), from at least one patient with endometrial cancer of type I, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 patient(s) with endometrial cancer of type I. It is more preferred that the reference level is the level determined by measuring between 2 and 500 reference biological samples from between 2 and 500 patients with endometrial cancer of type I. It is even more preferred that the reference level is determined by measuring between 50 and 500 reference biological samples from between 50 and 500 patients with endometrial cancer of type I. It is most preferred that the reference level is determined by measuring between 100 and 500 reference biological samples from between 100 and 500 patients with endometrial cancer of type I.

It is also (additionally or alternatively) particularly preferred that the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference biological sample(s), from at least one patient with endometrial cancer of type II, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 patient(s) with endometrial cancer of type II. It is more preferred that the reference level is the level determined by measuring between 2 and 500 reference biological samples from between 2 and 500 patients with endometrial cancer of type II. It is even more preferred that the reference level is determined by measuring between 50 and 500 reference biological samples from between 50 and 500 patients with endometrial cancer of type II. It is most preferred that the reference level is determined by measuring between 100 and 500 reference biological samples from between 100 and 500 patients with endometrial cancer of type II.

It is practicable to take one reference biological sample per individual for analysis. If additional reference biological samples are required, e.g. to determine the reference level in different reference biological samples, the same individual may be (re)tested. Said reference level may be an average reference level. It may be determined by measuring reference levels and calculating the "average" value (e.g. mean, median or modal value) thereof.

It is further preferred that
(i) the reference level is the level determined by measuring at least one reference biological sample from at least one patient with endometrial cancer of type I,
   wherein the level of eIF4g above the reference level indicates that the individual has endometrial cancer of type II,
(ii) the reference level is the level determined by measuring at least one reference biological sample from at least one patient with endometrial cancer of type II,
   wherein the level of the at least one eIF below the reference level indicates that the individual has endometrial cancer of type I.

Preferably, the level of the at least one elF is at least 0.6-fold or 0.7-fold, more preferably at least 0.8-fold or 0.9-fold, even more preferably at least 1.2-fold or 1.5-fold, and most preferably at least 2.0-fold or 3.0-fold above/below the reference level. For example, the level of the at least one elF is at least 0.6-fold, at least 0.7-fold, at least 0.8-fold, at least 0.9-fold, at least 1.0-fold, at least 1. 1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2. 1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold above/below the reference level.

The endometrial cancer of type I can have grade/stage 1, 2, or 3. Endometrial cancer of type II has usually grade/stage 3. Preferably, the endometrial cancer of type I is endometrioid cancer of grades 1 and 2. Preferably, the endometrial cancer of type II is uterine serous carcinomas, clear cell carcinomas or endometrioid cancer of grade 3.

In the methods as described herein, it is preferred that the biological sample is a tissue sample, e.g. tumor tissue sample, or a body fluid sample. It is also preferred that the reference biological sample is a tissue sample, e.g. tumor tissue sample, or a body fluid sample. Preferably, the body fluid sample is selected from the group consisting of a blood sample, an urine sample, a lymph sample, a saliva sample and a combination thereof. More preferably, the blood sample is a whole blood sample or a blood fraction sample. Even more preferably, the blood fraction sample is a blood serum sample, a blood plasma sample or a blood cell sample.

Preferably, the aforementioned samples are pre-treated before they are used in the methods described herein. Said pre-treatment may include treatments required to separate the at least one elF described herein, or to remove excessive material or waste. Furthermore, pre-treatments may aim at sterilizing samples and/or removing contaminants such as undesired cells, bacteria or viruses. Suitable techniques comprise centrifugation, extraction, fractioning, ultrafiltration, protein precipitation followed by filtration and purification and/or enrichment of compounds. Moreover, other pre-treatments are carried out in order to provide the at least one eIF described herein in a form or concentration suitable for analysis.

In one preferred embodiment of the methods described herein, the biological sample used to determine the level of the at least one elF is a tissue sample, e.g. tumor tissue sample (obtainable e.g. by biopsy) or a body fluid sample. The elF markers described herein can be found in the tissue affected with the tumor and in body fluids like blood and blood components (e.g. plasma or serum).

According to another preferred embodiment of the methods described herein, the level of the at least one elF is determined by measuring mRNA or protein levels. The levels of the eIFs in the methods described herein can be determined either by measuring mRNA molecules encoding said eIFs or the eIFs as such in form of proteins. Methods to determine mRNA levels and protein levels in a sample are well known. mRNA expression levels are usually measured by polymerase chain reaction (PCR), in particular by reverse transcription quantitative polymerase chain reaction (RT-PCR and qPCR) or real-time PCR. RT-PCR is used to create a cDNA from the mRNA. The cDNA may be used in a qPCR assay to produce fluorescence as the DNA amplification process progresses. This fluorescence is proportional to the original mRNA amount in the samples. Other methods to be used include Northern blots, Fluorescence in situ hybridization (FISH), microarrays, and RT-PCR combined with capillary electrophoresis. Protein levels of eIFs are preferably determined using immunoassays. Such methods are based on the binding of an antibody, a derivative or a fragment thereof to its corresponding target (i.e. eIF). Polyclonal and monoclonal antibodies can be used in such methods. Derivatives or fragments of antibodies include Fab fragments, F(ab')₂ fragments, Fv fragments, single chain antibodies and single domain antibodies. Preferred immunoassays include Western blot, Immunohistochemistry, ELISA (enzyme-linked immunosorbent assay), radioimmunoassays, fluorescence resonance energy transfer (FRET) or time resolved-FRET (TR-FRET). Immunoassays detection is possible in lymphoma and HCC. It is particularly preferred to use antibodies and derivatives or fragments of antibodies which have been obtained from a non-human source. These antigen binding molecules can be of porcine, rabbit, murine, camel or rat origin. Of course, it is also possible to use antibodies and derivatives or fragments thereof which are recombinantly produced in plants or cell cultures, in particular microbial cell cultures (e.g. bacteria, yeast).

In a third aspect, the present invention relates to the (*in vitro*) use of eIF6 for diagnosing endometrial cancer in an individual (suspected of having endometrial cancer).

For the above-mentioned use, the level of eIF6 is determined/analyzed in a biological sample from an individual. Thus, in a preferred embodiment, the present invention relates to the *(in vitro)* use of eIF6 for diagnosing endometrial cancer in an individual (suspected of having endometrial cancer) by determining/analyzing the level of eIF6 in a biological sample from said individual.

Preferred combinations with eIF6 can be taken from **Figure 6****.**

eIF4g and/or eIF2α may further be used, i.e. in addition to eIF6, for this purpose. In this respect, it should be noted that eIF4g preferably comprises the isoforms eIF4g1, eIF4g2, and/or eIF4g3. In addition, peIF2α is the phosphorylated form of eIF2α.

Preferred combinations with eIF6 can also be taken from **Figure 6****.**

It is preferred that the biological sample is a tissue sample, e.g. tumor tissue sample, or a body fluid sample. It is also preferred that the reference biological sample is a tissue sample, e.g. tumor tissue sample, or a body fluid sample. Preferably, the body fluid sample is selected from the group consisting of a blood sample, an urine sample, a lymph sample, a saliva sample, and a combination thereof. More preferably, the blood sample is a whole blood sample or a blood fraction sample. Even more preferably, the blood fraction sample is a blood serum sample, a blood plasma sample or a blood cell sample.

It is also (alternatively or additionally) preferred that the endometrial cancer is selected from the group consisting of endometrioid cancer, uterine serous carcinomas, and clear cell carcinomas. Endometrial cancer can further be distinguished into two main types, namely endometrial cancer of type I (EC I) and endometrial cancer of type II (EC II). Thus, it is more preferred that the endometrial cancer is endometrial cancer of type I or endometrial cancer of type II. It is even more preferred that the endometrial cancer of type I is endometrioid cancer of grades 1 and 2. It is even more preferred that the endometrial cancer of type II is uterine serous carcinomas, clear cell carcinomas or endometrioid cancer of grade 3.

As to further preferred embodiments, it is referred to the first aspect of the present invention.

Further described herein, but not encompassed by the present invention, is the *(in vitro)* use of eIF5 for determining the course of endometrial cancer in an individual (suffering from endometrial cancer).

For the above-mentioned use, the level of eIF5 is determined/analyzed in a biological sample from an individual. Thus, in a preferred embodiment, the *(in vitro)* use of eIF5 for determining the course of endometrial cancer in an individual (suffering from endometrial cancer) by determining/analyzing the level of eIF5 in a biological sample from said individual is described.

It is preferred that the biological sample is a tissue sample, e.g. tumor tissue sample, or a body fluid sample. It is also preferred that the reference biological sample is a tissue sample, e.g. tumor tissue sample, or a body fluid sample. Preferably, the body fluid sample is selected from the group consisting of a blood sample, an urine sample, a lymph sample, a saliva sample and a combination thereof. More preferably, the blood sample is a whole blood sample or a blood fraction sample. Even more preferably, the blood fraction sample is a blood serum sample, a blood plasma sample or a blood cell sample.

It is also (alternatively or additionally) preferred that the endometrial cancer is selected from the group consisting of endometrioid cancer, uterine serous carcinomas, and clear cell carcinomas. Endometrial cancer can further be distinguished into two main types, namely endometrial cancer of type I (EC I) and endometrial cancer of type II (EC II). Thus, it is more preferred that the endometrial cancer is endometrial cancer of type I or endometrial cancer of type II. It is even more preferred that the endometrial cancer of type I is endometrioid cancer of grades 1 and 2. It is even more preferred that the endometrial cancer of type II is uterine serous carcinomas, clear cell carcinomas or endometrioid cancer of grade 3.

As to further preferred embodiments, it is referred to the above.

In a fourth aspect, the present invention relates to the (*in vitro*) use of eIF6 for grading endometrial cancer in an individual (suffering from endometrial cancer).

For the above-mentioned use, the level of eIF6 is determined/analyzed in a biological sample from an individual. Thus, in a preferred embodiment, the present invention relates to the (*in vitro*) use of eIF6 for grading endometrial cancer in an individual (suffering from endometrial cancer) by determining/analyzing the level of eIF6 in a biological sample from said individual.

It may be determined that the individual suffers from endometrial cancer of grades 1, 2, or 3.

It is preferred that the biological sample is a tissue sample, e.g. tumor tissue sample, or a body fluid sample. It is also preferred that the reference biological sample is a tissue sample, e.g. tumor tissue sample, or a body fluid sample. Preferably, the body fluid sample is selected from the group consisting of a blood sample, an urine sample, a lymph sample, a saliva sample and a combination thereof. More preferably, the blood sample is a whole blood sample or a blood fraction sample. Even more preferably, the blood fraction sample is a blood serum sample, a blood plasma sample or a blood cell sample.

It is also (alternatively or additionally) preferred that the endometrial cancer is selected from the group consisting of endometrioid cancer, uterine serous carcinomas, and clear cell carcinomas. Endometrial cancer can further be distinguished into two main types, namely endometrial cancer of type I (EC I) and endometrial cancer of type II (EC II). Thus, it is more preferred that the endometrial cancer is endometrial cancer of type I or endometrial cancer of type II. It is even more preferred that the endometrial cancer of type I is endometrioid cancer of grades 1 and 2. It is even more preferred that the endometrial cancer of type II is uterine serous carcinomas, clear cell carcinomas or endometrioid cancer of grade 3.

When the histological type of endometrial cancer is endometrioid, the grade may be 1, 2 or 3. When the histological type is uterine serous or clear cell carcinoma, the grade is usually 3 and carries a worse prognosis. Thus, it may be determined that the individual suffers from endometrioid of grades 1, 2, or 3. It may also be determined that the individual suffers from uterine serous or clear cell carcinoma of grade 3.

As to further preferred embodiments, it is referred to the second aspect of the present invention.

Further described herein, but not encompassed by the present invention, is the *(in vitro)* use of eIF4g for differentiating between endometrial cancer of type I and endometrial cancer of type II in an individual (suffering from endometrial cancer).

In this respect, it should be noted that eIF4g preferably comprises the isoforms eIF4g1, eIF4g2, and/or eIF4g3.
For the above-mentioned use, the level of eIF4g is determined/analyzed in a biological sample from an individual to be tested. Thus, the (*in vitro*) use of eIF4g for differentiating between endometrial cancer of type I and endometrial cancer of type II in an individual (suffering from endometrial cancer) by determining/analyzing the level of eIF4g in a biological sample from said individual is described.

It is preferred that the biological sample is a tissue sample, e.g. tumor tissue sample, or a body fluid sample. It is also preferred that the reference biological sample is a tissue sample, e.g. tumor tissue sample, or a body fluid sample. Preferably, the body fluid sample is selected from the group consisting of a blood sample, an urine sample, a lymph sample, a saliva sample and a combination thereof. More preferably, the blood sample is a whole blood sample or a blood fraction sample. Even more preferably, the blood fraction sample is a blood serum sample, a blood plasma sample or a blood cell sample.

The endometrial cancer of type I can have grade/stage 1, 2, or 3. Endometrial cancer of type II has usually grade/stage 3. Preferably, the endometrial cancer of type I is endometrioid cancer of grades 1 and 2. Preferably, the endometrial cancer of type II is uterine serous carcinomas, clear cell carcinomas or endometrioid cancer of grade 3.

As to further preferred embodiments, it is referred to the above.

In a fifth aspect, the present invention relates to the use of a kit for diagnosing or grading endometrial cancer in an individual, wherein said kit comprises means for determining the level of eIF6 in a biological sample from an individual.

Preferred combinations with eIF6 can be taken from **Figure 6****.** Means for determining the level of eIF4g and/or eIF2α may further be comprised in the kit, i.e. in addition to the means for determining the level of eIF6. In this respect, it should be noted that eIF4g preferably comprises the isoforms eIF4g1, eIF4g2, and/or eIF4g3. In addition, peIF2α is the phosphorylated form of eIF2α. Preferred combinations with eIF6 can also be taken from **Figure 6****.**

Said means may be primers or primer pairs allowing the detecting of the above-mentioned eIFs on the RNA transcript, e.g. mRNA, level and/or antibodies, antibody derivatives or fragments of antibodies allowing the detection of the above-mentioned eIFs on the protein level.

In addition, said means encompass dipstrips or dipsticks, e.g. urine or blood dipstrips or dipsticks. Said means are tools used to determine changes in individual's urine or blood. A dipstrip or dipstick comprises different chemical pads or reagents which react (e.g. change color, in particular by applying an immune assay) when immersed in (e.g. blood or urine), and then removed from the biological sample (e.g. urine or blood sample). The result can be read after a few minutes, preferably after a few seconds.

The kit is preferably useful for conducting the methods according to the first and second aspect of the present invention.

Preferably, the kit further comprises
(i) a container, and/or
(ii) a data carrier.

Said data carrier may be a non-electronical data carrier, e.g. a graphical data carrier such as an information leaflet, an information sheet, a bar code or an access code, or an electronical data carrier such as a floppy disk, a compact disk (CD), a digital versatile disk (DVD), a microchip or another semiconductor-based electronical data carrier. The access code may allow the access to a database, e.g. an internet database, a centralized, or a decentralized database. The access code may also allow access to an application software that causes a computer to perform tasks for computer users or a mobile app which is a software designed to run on smartphones and other mobile devices.

Said data carrier may further comprise a reference level of eIF6.

In case that the data carrier comprises an access code which allows the access to a database, said reference level is deposited in this database.

In addition, the data carrier may comprise information or instructions on how to carry out the methods according to the first and second aspect of the present invention.

Said kit may also comprise materials desirable from a commercial and user standpoint including a buffer(s), a reagent(s) and/or a diluent(s) for determining the level mentioned above.

It is preferred that the biological sample is a tissue sample, e.g. tumor tissue sample, or a body fluid sample. It is also preferred that the reference biological sample is a tissue sample, e.g. tumor tissue sample, or a body fluid sample. Preferably, the body fluid sample is selected from the group consisting of a blood sample, an urine sample, a lymph sample, a saliva sample and a combination thereof. More preferably, the blood sample is a whole blood sample or a blood fraction sample. Even more preferably, the blood fraction sample is a blood serum sample, a blood plasma sample or a blood cell sample.

It is also (alternatively or additionally) preferred that the endometrial cancer is selected from the group consisting of endometrioid cancer, uterine serous carcinomas, and clear cell carcinomas. Endometrial cancer can further be distinguished into two main types, namely endometrial cancer of type I (EC I) and endometrial cancer of type II (EC II). Thus, it is more preferred that the endometrial cancer is endometrial cancer of type I or endometrial cancer of type II. It is even more preferred that the endometrial cancer of type I is endometrioid cancer of grades 1 and 2. It is even more preferred that the endometrial cancer of type II is uterine serous carcinomas, clear cell carcinomas or endometrioid cancer of grade 3.

### BRIEF DESCRIPTION OF THE FIGURES

The following Figures are merely illustrative of the present invention and should not be construed to limit the scope of the invention as indicated by the appended claims in any way.
**Figure 1****:** eIF protein expression in endometrial carcinoma (EC) compared to non-neoplastic tissue (NNT) was analyzed using immunohistochemistry. Alterations in protein expression pattern of eIF2α, eIF3c, eIF5, eIF6, and eIF4g using combined score (CS). CS was stratified into low (<= 11 points) and high (> 11 points).
**Figure 2****:** eIF protein expression in endometrial carcinoma compared to non-neoplastic tissue (NNT) was analyzed using immunoblot (western blot) analysis. Alterations in protein expression pattern of peIF2α, eIF3c, and eIF4g. Densitometric analyses of immunoblots were performed using ImageJ software (NIH, MD, United States). Relative densities were normalized to the loading control (Actin). Statistical analyses: t test with Mann-Whitney *U* test ; *p< 0.05; **p< 0.01 and ***p< 0.001.
**Figure 3****:** eIF protein expression in endometrial carcinoma (EC) grade G1, G2, and G3 compared to non-neoplastic tissue (NNT) was analyzed using immunohistochemistry. eIF5 and eIF6 allow grading of individuals suffering from endometrial carcinoma. Combined scores (CS), divided into low (CS <=11) and high (CS > 11) are plotted against NNT, and G1-G3 EC.
**Figure 4****:** eIF4g protein expression depending on endometrial cancer (EC) type. eIF4g combined scores (CS) stratified into 4 groups for type I and II EC. eIF4g allows to differentiate between endometrial cancer of type I and endometrial cancer of type 2.
**Figure 5****:** Representative immunohistochemical staining of eIF4g in endometrial carcinoma. Left panel showing low cytoplasmic eIF4g staining. Right panel showing high cytoplasmic eIF4g staining (magnification: x10).
**Figure 6****:** Preferred eIFs and elF combinations/signatures.

### EXAMPLES

The examples given below are for illustrative purposes only and do not limit the invention described above in any way.

### 1. Material and Methods

### 1.1 Patient samples

279 patients diagnosed with EC between January 2000 and September 2010 at a single institution (*Medical University of Gdansk*) were included in this retrospective study. A total of 1180 FFPE samples from primary tumour were analysed, with an average of four samples deriving from each patient. 15 endometrial mucosa samples from healthy individuals served as the control group. Basic demographic and pathological features are listed in **Table 1.**

**Table 1: Characterisation of demographic and tumour-related parameters.**

| | | **Count** | **%** | **Missing** |
|---|---|---|---|---|
| **Age** | < *60 years* | 117 | 41.9 | 0 |
| | > *60 years* | 162 | 58.1 | |
| **EC Type** | *Type I* | 216 | 90.0 | 39 |
| | *Type II* | 24 | 10.0 | |
| **Grading** | *G1* | 134 | 48.0 | 0 |
| | *G2* | 107 | 38.4 | |
| | *G3* | 38 | 13.6 | |
| **Staging** | *I* | 184 | 67.4 | 5 |
| | *II* | 42 | 15.2 | |
| | *III* | 34 | 12.3 | |
| | *IV* | 14 | 5.1 | |
| **Healthy control group** | | 9 | 100.0 | 0 |

### 1.2 Construction of TMA

Tumours were reviewed by two experienced observers using haematoxylin and eosin stained sections, and relevant tumour areas were marked on the slide. Tissue arrays of 1.5 mm in diameter were punched out from the chosen tumour areas and embedded into a fresh paraffin block according to a specific pattern. Tissue sections were cut at 4 µm and mounted on specific adhesive-coated glass slides compatible for immunohistochemical staining.

Tumour type and grade were histologically diagnosed according to the current WHO classification (11), the tumour stage according to UICC. Grade 3 EC was automatically classified as type II EC.

### 1.3 Immunohistochemistry (IHC)

IHC was performed on a Ventana Immunostainer XT (*Ventana Medical Systems, Tucson, USA*), using an ultra-VIEW universal DAB Detection Kit (*Ventana Medical Systems, Tucson, USA*) and cell conditioning solution for 30 minutes using heat induced epitope retrieval (HIER). The primary antibodies were incubated for 30 minutes using different dilutions (**Table 2**)**.**

**Table 2: Dilutions of antibodies used for eIF2a, eIF3c, eIF4g, eIF5, and eIF6 immunostaining.**

| **Primary Antibody** | **Company** | **Dilution** | **Second Antibody** |
|---|---|---|---|
| eIF4g | Cell Signaling (#2498) | 1:25 | Rabbit |
| eIF2α (D7D3) XP | Cell Signaling (#5324) | 1:2000 | Rabbit |
| eIF3c | Santa Cruz (sc-74507) | 1:250 | Mouse |
| eIF6 | Gene Tex (GTX63642) | 1:75 | Rabbit |
| eIF5 | Santa Cruz (sc-133541) | 1:50 | Rabbit |

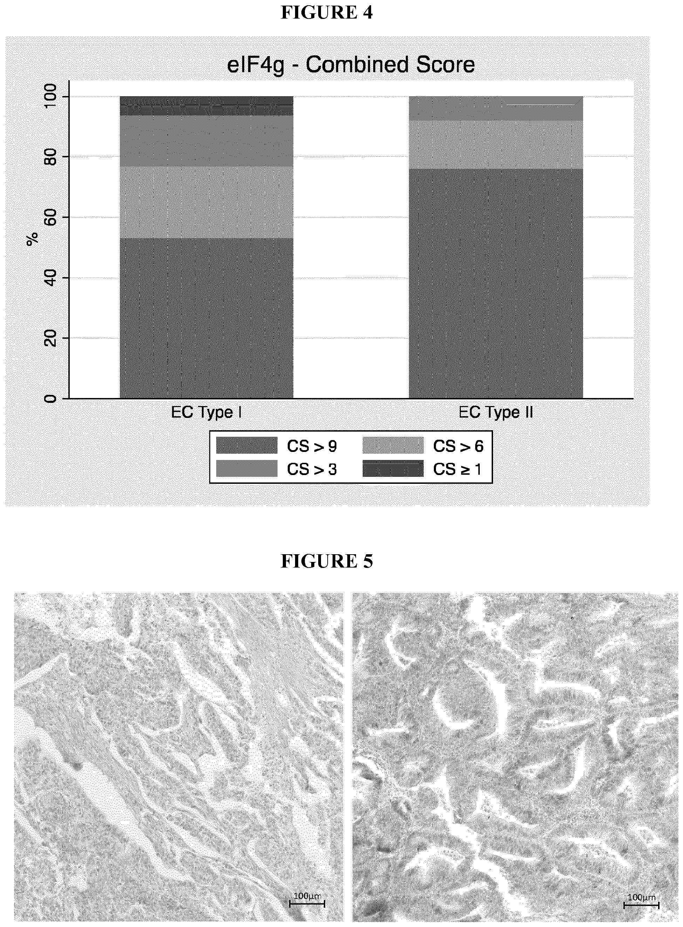

On average, four samples of each tumour were analysed. The TMAs were scored by a research assistant (M.A.S.) and a consultant pathologist (J.H.) using light microscopy. For each sample, immunostaining of eIF2a, eIF3c, eIF4g, eIF5, and eIF6 was assessed (exemplarily shown for eIF4g, see **Figure 5**). Based on visual estimation, cytoplasmic staining intensity was scored from zero to three (0=negative; 1=weak; 2=moderate; 3=strong). Cytoplasmic staining density was classified from one to four, based on the percentage of stained cells (0%-25% =1; 25%-50% =2, 50%-75% =3; 75%-100% =4). A combined score (CS) was subsequently calculated by multiplying staining density and intensity, resulting in a maximum value of 12. In case of poor staining quality of a certain sample, it was labelled as "non-classifiable" and excluded from statistical analysis.

### 1.4 Protein extraction and immunoblot

All tumor tissue samples were acquired during surgery, immediately frozen in liquid nitrogen and stored at -80°C. Frozen tissue samples were homogenized with a MagNA Lyser homogenizer (Roche Diagnostics, Risch-Rotkreuz, Switzerland) and lysed in NP-40 Lysis buffer (0.05 M Tris-HCl, 5 mM NaCl, 0.5% NP-40, 0.1 mM Pefabloc, 1 mM DTT, complete Mini, PhosSTOP). The protein concentration was determined using Bradford protein assay (Biorad Protein Assay Dye Reagent, 500-0006; BioRad Laboratories GmbH, Munich, Germany). Equal amounts of 30 µg protein were loaded onto SDS-PAGE gels (30% Acrylamid/ Bisacrylamid solution; ROTH, Karlsruhe, Germany), subjected to electrophoresis in Mini-vertical electrophoresis units (Hoefer Inc, Richmond, USA) and blotted onto PVDF membranes (Immobilin-P Transfer Membrane; Millipore, Massachusetts, USA) using a Semi Dry Blotting Unit (SCIE-PLAS; Cambridge, England). The membranes were blocked in TBS tween (TBST) with 5% non-fat milk (AppliChem; Darmstadt, Germany) for 1h at room temperature. The primary antibodies (**Table 3**) were diluted in TBST, 5% BSA and applied overnight at 4°C. The membranes were washed with TBST, followed by incubation with a horseradish peroxidase conjugated secondary antibody (anti-rabbit 1:5000; GE Healthcare Life Sciences, Buckinghamshire, England). Proteins were visualized using a chemiluminescence ECL kit (GE Healthcare Life Sciences), followed by exposure on the Image Quant LAS 500 (GE Healthcare, Little Chalfont, UK). The signal was normalized using anti-β-actin antibody (mAb dilution 1: 1000, Sigma-Aldrich, Missouri, USA).

**Table 3: Dilutions of antibodies used for peIF2a, eIF3c, and eIF4g immunoblot.**

| **Primary Antibody** | **Company** | **Dilution** | **Secondary Antibody** |
|---|---|---|---|
| Anti-Actin | Sigma (A2103) | 1:1000 | Rabbit |
| Phospho-eIF2α (Ser51)(D9G8) | Cell Signaling (#3398) | 1:1000 | Rabbit |
| eIF3c | Cell Signaling (#2068) | 1:1000 | Rabbit |
| eIF4g | Cell Signaling (#2498) | 1:1000 | Rabbit |

### 1.5 Statistical Analysis

Statistical analysis was carried out using SPSS Version 22.0 (IBM Statistics). Demographic and tumour-related information was summarised with descriptive and explorative analysis. The mean value of all samples deriving from one patient (4 samples on average) was calculated and used for statistical analysis, resulting in 279 cases.

CS for every elF was subdivided into "low" (value <=11) and "high" (value >11) staining groups. Chi-squared tests were used to determine differences between groups and CS staining groups. Means in eIF CS between two groups were compared with t-tests, and with one-way analysis of variance (ANOVA) between >2 groups, with post-hoc t-tests for comparison between the individual groups.

### 1.6 Ethics Statement

The study was accepted by the Independent Ethics Committee of the Medical University of Gdańsk (NKEBN/269/2009). Procedures involving human subjects were in accordance with the Helsinki Declaration of 1975, as revised in 1983.

### 2. Results

### 2.1 Immunohistochemistry

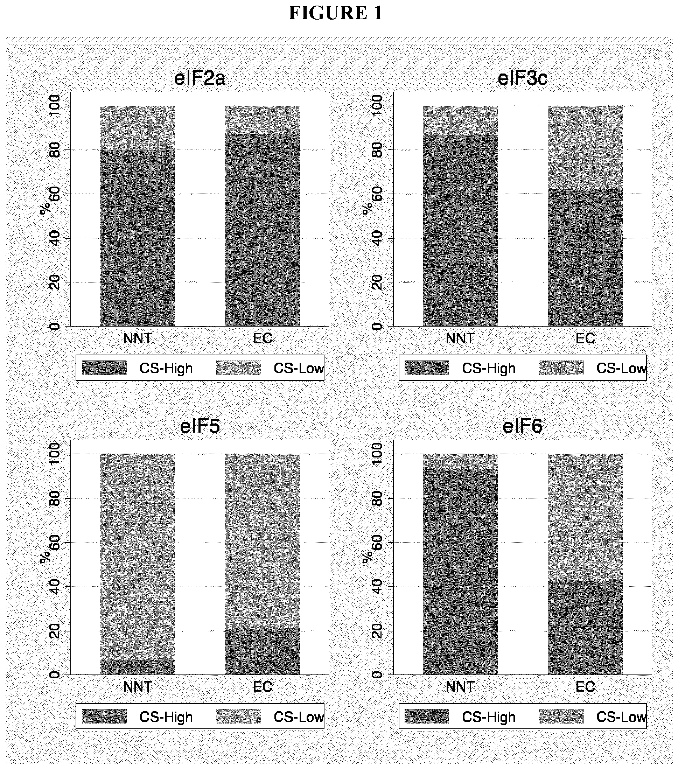

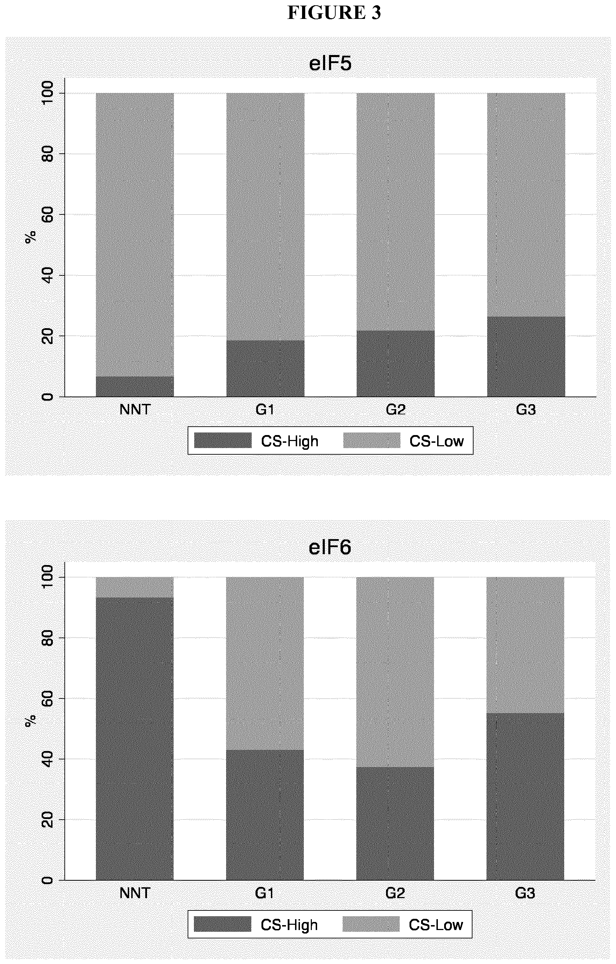

The tested eIFs showed a strong correlation among each other (**data not shown**). There was a significant difference in eIF-expression between tumour tissue and non-neoplastic tissue (NNT). The mean CS of eIF2α was 10.7 points for control samples (NNT) and 11.7 points for tumour samples (T-test; p=0.004). Thus, the mean CS of eIF2α was higher in tumours as compared to NNT. Likewise, the mean eIF5 CS was higher in tumours as compared to NNT (7.7 vs 4.6; T-test; p=0.002) and did also significantly differ between NNT and grading (one-way ANOVA, p<0.001; post-hoc t-test; NNT vs. G1, p=0.001; NNT vs. G2, p<0.001; NNT vs. G3, p<0.001; G1 vs. G2, p=0.203; G1 vs. G3, p=0.021; G2 vs. G3, p=0.166; **Figure 3**, CS split into "low" and "high" for viewing purposes). The mean CS of eIF6 was also different between tumour samples and NNT (9.5 *vs.* 11.6; T-test; p=0.004). Low eIF6 CS was more frequent in tumour tissue as compared to NNT and slightly increased with ongoing dedifferentiation (one-way ANOVA; p=0.008; post-hoc t-tests: NNT vs. G1, p=0.005; NNT vs. G2, p=0.002; NNT vs. G3, p=0.094; G1 vs. G2, p=0.495; G1 vs. G3, p=0.150; G2 vs. G3, p=0.062; **Figure 3**, CS split into "low" and "high" for viewing purposes). eIF4g CS was significantly higher in type II EC as compared to type I EC (10.4 vs. 8.9; T-test; p=0.022; **Figure 4**). The mean eIF3c CS expression was also different between NNT and EC (10.8 vs. 10.4). These results are shown in **Figure 1**, with CS split into "low" (CS <= 11) and "high" (CS > 11) for viewing purposes.

Chi-squared tests were further made to evaluate differences between CS and clinico-pathological parameters.

From the data presented in **Figure 3**, it can be concluded that eIF6 and eIF5 allow the determination of the grade of the endometrial carcinoma. Furthermore, separating staining into "low" and "high" allows for differentiation between NNT, and G1, G2 and G3 EC (**Table 4**), whilst this effect is not evident for eIF5 when separated into "low" and "high" staining group.

**Table 4: Chi-squared tests evaluating differences between CS and clinico-pathological parameters.**

| | | **eIF5** | | | | **eIF6** | | |
|---|---|---|---|---|---|---|---|---|
| | | **Low** | **High** | **p-value** | | **Low** | **High** | **p-value** |
| **Age** | < *60 years* | 97 | 23 | 0.552 | | 61 | 59 | 0.058 |
| | > *60 years* | 129 | 37 | | | 103 | 63 | |
| **Type** | *Type I* | 183 | 37 | 0.168 | | 131 | 89 | 0.468 |
| | *Type II* | 18 | 7 | | | 13 | 12 | |
| **Grading** | *Control* | 8 | 1 | 0.390 | | 1 | 8 | < 0.001 |
| | *G1* | 110 | 25 | | | 77 | 58 | |
| | G2 | 86 | 24 | | | 69 | 41 | |
| | *G3* | 28 | 11 | | | 17 | 22 | |
| **Staging** | *Control* | 8 | 1 | 0.156 | | 1 | 8 | 0.087 |
| | *I* | 145 | 42 | | | 106 | 81 | |
| | *II* | 38 | 7 | | | 28 | 17 | |
| | *III* | 30 | 4 | | | 19 | 15 | |
| | *IV* | 9 | 6 | | | 8 | 7 | |

### 2.2 Immunoblot (western blot) analysis

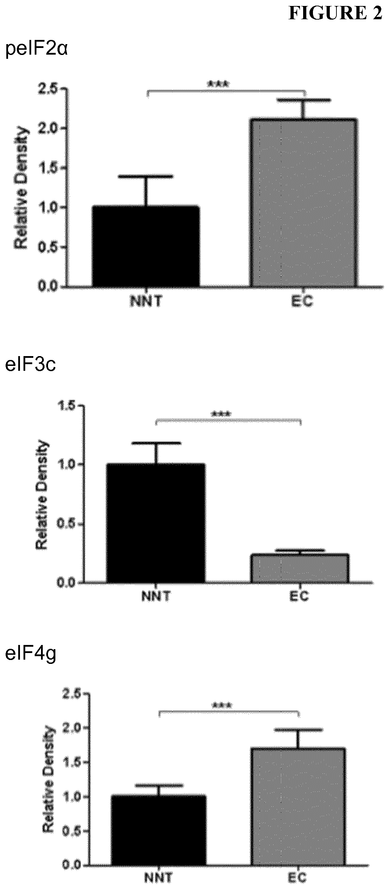

Thee expression patterns of peIF2α, eIF3c, and eIF4g were investigated in endometrial cancer compared to non-neoplastic patient samples using immunoblot analysis. The results are shown in **Figure 2****.** The protein expression levels of peIF2α and eIF4g were significantly increased in EC patients compared to NNT. The protein expression level of the eIF3c was significant decreased in EC compared to NNT.

### REFERENCES

1. Ferlay J, Soerjomataram I, Dikshit R, Eser S, Mathers C, Rebelo M, et al. Cancer incidence and mortality worldwide: sources, methods and major patterns in GLOBOCAN 2012. Int J Cancer 2015;136(5):E359-86.
2. Platz CE, Benda JA. Female genital tract cancer. Cancer 1995;75(1 Suppl):270-94.
3. Linkov F, Edwards R, Balk J, Yurkovetsky Z, Stadterman B, Lokshin A, et al. Endometrial hyperplasia, endometrial cancer and prevention: gaps in existing research of modifiable risk factors. Eur J Cancer 2008;44(12):1632-44.
4. Ueda SM, Kapp DS, Cheung MK, Shin JY, Osann K, Husain A, et al. Trends in demographic and clinical characteristics in women diagnosed with corpus cancer and their potential impact on the increasing number of deaths. Am J Obstet Gynecol 2008;198(2):218 e1-6.
5. Bokhman JV. Two pathogenetic types of endometrial carcinoma. Gynecol Oncol 1983; 15(1): 10-7.
6. Murali R, Soslow RA, Weigelt B. Classification of endometrial carcinoma: more than two types. Lancet Oncol 2014;15(7):e268-78.
7. Cancer Genome Atlas Research N, Kandoth C, Schultz N, Cherniack AD, Akbani R, Liu Y, et al. Integrated genomic characterization of endometrial carcinoma. Nature 2013;497(7447):67-73.
8. McConechy MK, Ding J, Cheang MC, Wiegand KC, Senz J, Tone AA, et al. Use of mutation profiles to refine the classification of endometrial carcinomas. J Pathol 2012;228(1):20-30.
9. Tashiro H, Isacson C, Levine R, Kurman RJ, Cho KR, Hedrick L. p53 gene mutations are common in uterine serous carcinoma and occur early in their pathogenesis. Am J Pathol 1997;150(1):177-85.
10. Salvesen HB, Haldorsen IS, Trovik J. Markers for individualised therapy in endometrial carcinoma. Lancet Oncol 2012;13(8):e353-61
11. Tavassoli FA, Devilee P. Pathology and Genetics of Tumours of the Breast and Genital Organs. Lyon: IARCPress; 2000.

## Claims

1. An in vitro method of diagnosing endometrial cancer in an individual comprising the step of:
determining the level of eukaryotic Initiation Factor 6 (eIF6) in a biological sample from an individual.

2. The method of claim 1, wherein the level of eIF6 is compared to a reference level of said eIF6,
wherein preferably the reference level of said eIF6 is the level determined by measuring at least one reference biological sample from at least one healthy individual, and wherein more preferably
the level of eIF6 which is below the reference level of said eIF6 indicates that the individual suffers from endometrial cancer.

3. An in vitro method of grading endometrial cancer in an individual comprising the step of:
determining the level of eukaryotic Initiation Factor 6 (eIF6) in a biological sample from an individual.

4. The method of claim 3, wherein the level of eIF6 is compared to a reference level of said eIF6,
wherein preferably the reference level of said eIF6 is the level determined by measuring at least one reference biological sample
from at least one healthy individual,
from at least one patient with endometrial cancer of grade 1,
from at least one patient with endometrial cancer of grade 2, and/or
from at least one patient with endometrial cancer of grade 3.

5. The method of claim 4, wherein
(i) the reference level of said eIF6 is the level determined by measuring at least one reference biological sample from at least one healthy individual,
wherein the level of eIF6 below the reference level of said eIF6 indicates that the individual has endometrial cancer of grade 1,
(ii) the reference level of said eIF6 is the level determined by measuring at least one reference biological sample from at least one patient with endometrial cancer of grade 2,
wherein the level of eIF6 above the reference level of said eIF6 indicates that the individual has endometrial cancer of grade 1, and/or
(iii) the reference level of said eIF6 is the level determined by measuring at least one reference biological sample from at least one patient with endometrial cancer of grade 3,
wherein the level of eIF6 below the reference level of said eIF6 indicates that the individual has endometrial cancer of grade 1, or
wherein
(i) the reference level of said eIF6 is the level determined by measuring at least one reference biological sample from at least one healthy individual,
wherein the level of eIF6 below the reference level of said eIF6 indicates that the individual has endometrial cancer of grade 2,
(ii) the reference level of said eIF6 is the level determined by measuring at least one reference biological sample from at least one patient with endometrial cancer of grade 1,
wherein the level of eIF6 below the reference level of said eIF6 indicates that the individual has endometrial cancer of grade 2, and/or
(iii) the reference level of said eIF6 is the level determined by measuring at least one reference biological sample from at least one patient with endometrial cancer of grade 3,
wherein the level of eIF6 below the reference level of said eIF6 indicates that the individual has endometrial cancer of grade 2, or
wherein
(i) the reference level of said eIF6 is the level determined by measuring at least one reference biological sample from at least one healthy individual,
wherein the level of eIF6 below the reference level of said eIF6 indicates that the individual has endometrial cancer of grade 3,
(ii) the reference level of said eIF6 is the level determined by measuring at least one reference biological sample from at least one patient with endometrial cancer of grade 1,
wherein the level of eIF6 above the reference level of said eIF6 indicates that the individual has endometrial cancer of grade 3, and/or
(iii) the reference level of said eIF6 is the level determined by measuring at least one reference biological sample from at least one patient with endometrial cancer of grade 2,
wherein the level of eIF6 above the reference level of said eIF6 indicates that the individual has endometrial cancer of grade 3.

6. The method of any one of claims 1 to 5, wherein the endometrial cancer is endometrial cancer of type I or endometrial cancer of type II.

7. In vitro use of eIF6 for diagnosing endometrial cancer in an individual.

8. In vitro use of eIF6 for grading endometrial cancer in an individual.

9. Use of a kit for diagnosing or grading endometrial cancer in an individual, wherein said kit comprises means for determining the level of eIF6 in a biological sample from the individual.

## Patentansprüche

1. In-vitro-Verfahren zur Diagnose von Endometriumkrebs bei einem Individuum, umfassend den Schritt:
Bestimmen des Levels des eukaryotischen Initiationsfaktors 6 (eIF6) in einer biologischen Probe von einem Individuum.

2. Verfahren nach Anspruch 1, wobei das Level von eIF6 mit einem Referenzlevel von eIF6 verglichen wird,
wobei bevorzugt das Referenzlevel von eIF6 das Level ist, das durch Messen von mindestens einer biologischen Referenzprobe von mindestens einem gesunden Individuum bestimmt wird, und
wobei besonders bevorzugt das Level von eIF6, das unter dem Referenzlevel von eIF6 liegt, anzeigt, dass das Individuum an Endometriumkrebs leidet.

3. In-vitro-Verfahren zur Einstufung von Endometriumkrebs bei einem Individuum, umfassend den Schritt:
Bestimmen des Levels des eukaryotischen Initiationsfaktors 6 (eIF6) in einer biologischen Probe von einem Individuum.

4. Verfahren nach Anspruch 3, wobei das Level von eIF6 mit einem Referenzlevel von eIF6 verglichen wird,
wobei bevorzugt das Referenzlevel von eIF6 das Level ist, das durch Messen von mindestens einer biologischen Referenzprobe
von mindestens einem gesunden Individuum,
von mindestens einem Patienten mit Endometriumkrebs des Grades 1,
von mindestens einem Patienten mit Endometriumkarzinom des Grades 2 und/oder
von mindestens einem Patienten mit Endometriumkarzinom des Grades 3 bestimmt wird.

5. Verfahren nach Anspruch 4, wobei
(i) das Referenzlevel von eIF6 das Level ist, das durch Messen von mindestens einer biologischen Referenzprobe von mindestens einem gesunden Individuum bestimmt wird,
wobei das Level von eIF6 unterhalb des Referenzlevels von eIF6 anzeigt, dass das Individuum Endometriumkrebs des Grades 1 hat,
(ii) das Referenzlevel von eIF6 das Level ist, das durch Messen von mindestens einer biologischen Referenzprobe von mindestens einem Patienten mit Endometriumkrebs des Grades 2 bestimmt wird,
wobei das Level von eIF6 oberhalb des Referenzlevels von eIF6 anzeigt, dass das Individuum Endometriumkrebs des Grades 1 hat, und/oder
(iii) das Referenzlevel von eIF6 das Level ist, das durch Messen von mindestens einer biologischen Referenzprobe von mindestens einem Patienten mit Endometriumkrebs des Grades 3 bestimmt wird,
wobei das Level von eIF6 unterhalb des Referenzlevels von eIF6 anzeigt, dass das Individuum Endometriumkrebs des Grades 1 hat, oder
wobei
(i) das Referenzlevel von eIF6 das Level ist, das durch Messen von mindestens einer biologischen Referenzprobe von mindestens einem gesunden Individuum bestimmt wird,
wobei das Level von eIF6 unterhalb des Referenzlevels von eIF6 anzeigt, dass das Individuum Endometriumkrebs des Grades 2 hat,
(ii) das Referenzlevel von eIF6 das Level ist, das durch Messen von mindestens einer biologischen Referenzprobe von mindestens einem Patienten mit Endometriumkrebs des Grades 1 bestimmt wird,
wobei das Level von eIF6 unterhalb des Referenzlevels von eIF6 anzeigt, dass das Individuum Endometriumkrebs des Grades 2 hat, und/oder
(iii) das Referenzlevel von eIF6 das Level ist, das durch Messen von mindestens einer biologischen Referenzprobe von mindestens einem Patienten mit Endometriumkrebs des Grades 3 bestimmt wird,
wobei das Level von eIF6 unterhalb des Referenzlevels von eIF6 anzeigt, dass das Individuum Endometriumkrebs des Grades 2 hat, oder
wobei
(i) das Referenzlevel von eIF6 das Level ist, das durch Messen von mindestens einer biologischen Referenzprobe von mindestens einem gesunden Individuum bestimmt wird,
wobei das Level von eIF6 unterhalb des Referenzlevels von eIF6 anzeigt, dass das Individuum Endometriumkrebs des Grades 3 hat,
(ii) das Referenzlevel von eIF6 das Level ist, das durch Messen von mindestens einer biologischen Referenzprobe von mindestens einem Patienten mit Endometriumkrebs des Grades 1 bestimmt wird,
wobei das Level von eIF6 oberhalb des Referenzlevels von eIF6 anzeigt, dass das Individuum Endometriumkrebs des Grades 3 hat, und/oder
(iii) das Referenzlevel von eIF6 das Level ist, das durch Messen von mindestens einer biologischen Referenzprobe von mindestens einem Patienten mit Endometriumkrebs des Grades 2 bestimmt wird,
wobei das Level von eIF6 oberhalb des Referenzlevels von eIF6 anzeigt, dass das Individuum Endometriumkrebs des Grades 3 hat.

6. Verfahren nach einem der Ansprüche 1 bis 5, wobei der Endometriumkrebs ein Endometriumkrebs vom Typ I oder Endometriumkrebs vom Typ II ist.

7. In vitro-Verwendung von eIF6 zur Diagnose von Endometriumkrebs bei einem Individuum.

8. In vitro-Verwendung von eIF6 zur Einstufung von Endometriumkrebs bei einem Individuum.

9. Verwendung eines Kits zur Diagnose oder Einstufung von Endometriumkrebs bei einem Individuum, wobei das Kit Mittel zum Bestimmen des Levels von eIF6 in einer biologischen Probe des Individuums umfasst.

## Revendications

1. Méthode in vitro de diagnostic du cancer de l'endomètre chez un sujet comprenant l'étape de :
détermination du taux de facteur d'initiation eucaryote 6 (eIF6) dans un échantillon biologique prélevé chez un sujet.

2. Méthode selon la revendication 1, ledit taux de eIF6 étant comparé à un taux de référence dudit eIF6,
de préférence, ledit taux de référence dudit eIF6 étant le taux déterminé par la mesure d'au moins un échantillon biologique de référence prélevé chez au moins un sujet en bonne santé, et,
plus préférablement
ledit taux de eIF6 qui est inférieur au taux de référence dudit eIF6 indiquant que le sujet souffre d'un cancer de l'endomètre.

3. Méthode in vitro de classification du cancer de l'endomètre chez un sujet comprenant l'étape de :
détermination du taux de facteur d'initiation eucaryote 6 (eIF6) dans un échantillon biologique prélevé chez un sujet.

4. Méthode selon la revendication 3, ledit taux de eIF6 étant comparé à un taux de référence dudit eIF6,
de préférence ledit taux de référence dudit eIF6 étant le taux déterminé par la mesure d'au moins un échantillon biologique de référence
prélevé chez au moins un sujet en bonne santé,
prélevé chez au moins une patiente atteinte d'un cancer de l'endomètre de grade 1,
prélevé chez au moins une patiente atteinte d'un cancer de l'endomètre de grade 2, et/ou
prélevé chez au moins une patiente atteinte d'un cancer de l'endomètre de grade 3.

5. Procédé selon la revendication 4,
(i) de préférence ledit taux de référence dudit eIF6 étant le taux déterminé par la mesure d'au moins un échantillon biologique de référence prélevé chez au moins un sujet en bonne santé,
ledit taux de eIF6 inférieur au taux de référence dudit eIF6 indiquant que le sujet est atteint d'un cancer de l'endomètre de grade 1,
(ii) ledit taux de référence dudit eIF6 étant le taux déterminé par la mesure d'au moins un échantillon biologique de référence prélevé chez au moins une patiente atteinte d'un cancer de l'endomètre de grade 2,
ledit taux de eIF6 supérieur au taux de référence dudit eIF6 indiquant que le sujet est atteint d'un cancer de l'endomètre de grade 1, et/ou
(iii) ledit taux de référence dudit eIF6 étant le taux déterminé par la mesure d'au moins un échantillon biologique de référence prélevé chez au moins une patiente atteinte d'un cancer de l'endomètre de grade 3,
ledit taux de eIF6 inférieur au taux de référence dudit eIF6 indiquant que le sujet est atteint d'un cancer de l'endomètre de grade 1, ou
(i) ledit taux de référence dudit eIF6 étant le taux déterminé par la mesure d'au moins un échantillon biologique de référence prélevé chez au moins un sujet en bonne santé,
ledit taux de eIF6 inférieur au taux de référence dudit eIF6 indiquant que le sujet est atteint d'un cancer de l'endomètre de grade 2,
(ii) ledit taux de référence dudit eIF6 étant le taux déterminé par la mesure d'au moins un échantillon biologique de référence prélevé chez au moins une patiente atteinte d'un cancer de l'endomètre de grade 1,
ledit taux de eIF6 inférieur au taux de référence dudit eIF6 indiquant que le sujet est atteint d'un cancer de l'endomètre de grade 2, et/ou
(iii) ledit taux de référence dudit eIF6 étant le taux déterminé par la mesure d'au moins un échantillon biologique de référence prélevé chez au moins une patiente atteinte d'un cancer de l'endomètre de grade 3,
ledit taux de eIF6 inférieur au taux de référence dudit eIF6 indiquant que le sujet est atteint d'un cancer de l'endomètre de grade 2, ou
(i) ledit taux de référence dudit eIF6 étant le taux déterminé par la mesure d'au moins un échantillon biologique de référence prélevé chez au moins un sujet en bonne santé,
ledit taux de eIF6 inférieur au taux de référence dudit eIF6 indiquant que l'individu est atteint d'un cancer de l'endomètre de grade 3,
(ii) ledit taux de référence dudit eIF6 étant le taux déterminé par la mesure d'au moins un échantillon biologique de référence prélevé chez au moins une patiente atteinte d'un cancer de l'endomètre de grade 1,
ledit taux de eIF6 supérieur au taux de référence dudit eIF6 indiquant que l'individu est atteint d'un cancer de l'endomètre de grade 3, et/ou
(iii) ledit taux de référence dudit eIF6 étant le taux déterminé par la mesure d'au moins un échantillon biologique de référence prélevé chez au moins une patiente atteinte d'un cancer de l'endomètre de grade 2,
ledit taux de eIF6 supérieur au taux de référence dudit eIF6 indiquant que le sujet est atteint d'un cancer de l'endomètre de grade 3.

6. Procédé selon l'une quelconque des revendications 1 à 5, ledit cancer de l'endomètre étant un cancer de l'endomètre de type I ou un cancer de l'endomètre de type II.

7. Utilisation in vitro de eIF6 pour diagnostiquer le cancer de l'endomètre chez un sujet.

8. Utilisation in vitro de eIF6 pour classer le cancer de l'endomètre chez un sujet.

9. Utilisation d'un kit pour diagnostiquer ou classer le cancer de l'endomètre chez un sujet, ledit kit comprenant des moyens pour déterminer le taux de eIF6 dans un échantillon biologique prélevé chez le sujet.
